# EUROPEAN PATENT APPLICATION

(11) **EP 4 644 421 A1**
(43) Date of publication of application: **05.11.2025**
(21) Application number: 23910665.1
(22) Date of filing: 26.12.2023
(51) Int. Cl.: C07K 16/28, C07K 19/00, A61K 39/395, A61K 38/17, A61K 47/55, A61P 35/00

(54) **ANTI-CD94 ANTIBODY AND USE THEREOF**

(30) Priority: 30.12.2022 CN 202211739469
(71) Applicant: Hefei TG Immunopharma Co., Ltd., Hefei, Anhui 230001 (CN)
(72) Inventor: CAO, Guoshuai, Hefei, Anhui 230001 (CN); CHENG, Ying, Hefei, Anhui 230001 (CN); LI, Yangyang, Hefei, Anhui 230001 (CN); WU, Yuwei, Hefei, Anhui 230001 (CN)
(74) Representative: Latscha Schöllhorn Partner AG
(86) International application number: PCT/CN2023/142132
(87) International publication number: WO 2024/140742

(57) **Abstract**

An anti-CD94 antibody and a use thereof. The antibody comprises heavy chain CDR1, CDR2, and CDR3, and light chain CDR1, CDR2, and CDR3, wherein: the amino acid sequences of the heavy chain CDR1, CDR2, and CDR3 respectively have at least 80% homology with the sequences represented by SEQ ID NOs 1, 2, and 3, and the amino acid sequences of the light chain CDR1, CDR2, and CDR3 respectively have at least 80% homology with the sequences represented by SEQ ID NOs 4, 5, and 6. The present antibody can bind to human CD94 proteins, relieve CD94/NKG2A-mediated immunosuppression, promote the activation of T cells, and can effectively treat and prevent diseases related to CD94 mediation.

## Description

### FIELD

The present disclosure relates to the field of biomedicine, and particularly, to an anti-CD94 antibody and use thereof.

### BACKGROUND

Cancer poses a severe threat to human life and health. In addition to traditional treatment methods such as surgical resection, radiotherapy, and chemotherapy, various cancer treatment methods such as small molecule targeted therapies, immune checkpoint therapies, cell and gene therapies are also under development.

In recent years, an immunotherapy represented by PD-1/L1 has demonstrated substantial potential, and three types of immunotherapies, namely PD-1/L1, CTLA-4, and LAG-3, have been approved as drugs for marketing. However, it cannot be ignored that even the PD-1/L1 therapy, which has the broadest range of approved indications, still has an overall response rate of only 30%, leaving a large number of patients unable to benefit from it.

Immune checkpoint molecules are inhibitory molecules expressed on surfaces of immune cells including T cells, NK cells, and monocyte-macrophages. After binding to corresponding ligands, they transmit inhibitory signals into the immune cells, inhibiting an anti-cancer function of the immune cells.

Due to significant heterogeneity in expressions of immune checkpoint receptors and ligands in tumors and tumor-infiltrating lymphocytes, a single type of the immune checkpoint therapy cannot be suitable for all patients, and most patients fail to benefit from it. On the other hand, some patients who have received the immune checkpoint therapy may experience tumor relapse and develop resistance to the immune checkpoint therapy, resulting in no therapeutic effects even with continued administration. For these two reasons, it is necessary to develop immune checkpoint antibodies against more targets.

CD94 is expressed on the surface of NK cells and T cells, and forms heterodimers with NKG2A and NKG2C. The ligand for all these heterodimers is HLA-E. Among them, CD94/NKG2A functions as an inhibitory receptor, while CD94/NKG2C functions as an activating receptor. However, tumor-infiltrating NK cells and T cells predominantly express CD94/NKG2A on their surface, rather than CD94/NKG2C. Therefore, immune checkpoint therapies targeting CD94 and NKG2A both hold potential value.

So far, there have been no public reports or clinical trials of the immune checkpoint therapy targeting CD94 worldwide. The most advanced therapy targeting NKG2A is a huZ270 antibody developed by Innate Pharma. Therefore, developing immune checkpoint therapies targeting CD94 is of great significance for treatment and prevention of diseases.

### SUMMARY

The present disclosure is provided based on the inventors' discovery of the following issues and facts.

CD94 is expressed on the surface of NK cells and T cells, and forms heterodimers with NKG2A and NKG2C. CD94/NKG2A is predominantly expressed on the surface of tumor-infiltrating NK cells and T cells and functions as an inhibitory receptor. Therefore, immune checkpoint therapies targeting CD94 and NKG2A hold potential value. However, there are not many tumor treatment methods and research approaches targeting a CD94 molecule remain limited.

The inventors of the present disclosure have successfully screened a murine anti-CD94 monoclonal antibody which has high binding activity to a human CD94 protein. In addition, the constant region of the above-mentioned monoclonal antibody is humanized and CDR of the murine anti-CD94 monoclonal antibody is retained, to obtain a chimeric antibody. Furthermore, the framework region in the light chain variable region or the heavy chain variable region of the chimeric antibody was humanized to obtain a fully humanized anti-CD94 antibody. The humanized antibody can specifically target and bind to the human CD94 protein, has characteristics of low immunogenicity, and can effectively treat and/or prevent a CD94-mediated disease. In the present disclosure, in vitro activities of the CD94 antibody and an NKG2A antibody huZ270 are compared. In a reporter system, the CD94 antibody exhibits a better activation of Jurkat T cells than the NKG2A antibody.

Therefore, in a first aspect of the present disclosure, the present disclosure provides an antibody or antigen-binding fragment thereof. According to embodiments of the present disclosure, the antibody or antigen-binding fragment thereof includes:
a heavy chain CDR1, a heavy chain CDR2, and a heavy chain CDR3; and
a light chain CDR1, a light chain CDR2, and a light chain CDR3, wherein:
   an amino acid sequence of the heavy chain CDR1 has at least 80% homology to a sequence as set forth in SEQ ID NO: 1;
   an amino acid sequence of the heavy chain CDR2 has at least 80% homology to a sequence as set forth in SEQ ID NO: 2;
   an amino acid sequence of the heavy chain CDR3 has at least 80% homology to a sequence as set forth in SEQ ID NO: 3;
   an amino acid sequence of the light chain CDR1 has at least 80% homology to a sequence as set forth in SEQ ID NO: 4;
   an amino acid sequence of the light chain CDR2 has at least 80% homology to a sequence as set forth in SEQ ID NO: 5; and
   an amino acid sequence of the light chain CDR3 has at least 80% homology to a sequence as set forth in SEQ ID NO: 6.

The antibody or antigen-binding fragment thereof according to the embodiments of the present disclosure can bind to the human CD94 protein, thereby effectively treating or preventing a CD94-mediated disease and being used for related scientific research.

According to an embodiment of the present disclosure, the above-mentioned antibody or antigen-binding fragment thereof may further include at least one of the following additional technical features.

According to an embodiment of the present disclosure, the antibody or antigen-binding fragment thereof includes at least one of a heavy chain FR region and a light chain FR region.

According to an embodiment of the present disclosure, at least part of the at least one of the heavy chain FR region and the light chain FR region is from at least one of a humanized antibody, a primate-derived antibody, a murine antibody, or a mutant thereof.

According to an embodiment of the present disclosure, the antibody or antigen-binding fragment thereof includes: at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 7 to SEQ ID NO: 10, respectively; or at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 15 to SEQ ID NO: 18, respectively.

According to an embodiment of the present disclosure, the antibody or antigen-binding fragment thereof includes: at least one of a light chain framework region LFR1 sequence, a light chain framework region LFR2 sequence, a light chain framework region LFR3 sequence, and a light chain framework region LFR4 sequence as set forth in SEQ ID NO: 11 to SEQ ID NO: 14, respectively; or at least one of a light chain framework region LFR1 sequence, a light chain framework region LFR2 sequence, a light chain framework region LFR3 sequence, and a light chain framework region LFR4 sequence as set forth in SEQ ID NO: 19 to SEQ ID NO: 22, respectively.

According to an embodiment of the present disclosure, the antibody or antigen-binding fragment thereof includes:
at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 7 to SEQ ID NO: 10, respectively, and at least one of a light chain framework region LFR1 sequence, a light chain framework region LFR2 sequence, a light chain framework region LFR3 sequence, and a light chain framework region LFR4 sequence as set forth in SEQ ID NO: 11 to SEQ ID NO: 14, respectively; or
at least one of a heavy chain framework region HFR1 sequence, a heavy chain framework region HFR2 sequence, a heavy chain framework region HFR3 sequence, and a heavy chain framework region HFR4 sequence as set forth in SEQ ID NO: 15 to SEQ ID NO: 18, respectively, and at least one of a light chain framework region LFR1 sequence, a light chain framework region LFR2 sequence, a light chain framework region LFR3 sequence, and a light chain framework region LFR4 sequence as set forth in SEQ ID NO: 19 to SEQ ID NO: 22, respectively.

According to an embodiment of the present disclosure, the antibody or antigen-binding fragment thereof includes a heavy chain variable region as set forth in SEQ ID NO: 23 or SEQ ID NO: 25; and/or a light chain variable region as set forth in SEQ ID NO: 24 or SEQ ID NO:26.

According to an embodiment of the present disclosure, the antibody or antigen-binding fragment thereof includes 1) a heavy chain variable region as set forth in SEQ ID NO: 23 and a light chain variable region as set forth in SEQ ID NO: 24; or 2) a heavy chain variable region as set forth in SEQ ID NO: 25 and a light chain variable region as set forth in SEQ ID NO: 26.

According to an embodiment of the present disclosure, the antibody or antigen-binding fragment thereof includes at least one of a heavy chain constant region and a light chain constant region, wherein at least part of the at least one of the heavy chain constant region and the light chain constant region is from at least one of a humanized antibody, a primate-derived antibody, a murine antibody, or a mutant thereof.

According to an embodiment of the present disclosure, the light chain constant region and the heavy chain constant region are each from: a murine IgG antibody or a mutant thereof; or a humanized IgG antibody or a mutant thereof.

According to an embodiment of the present disclosure, the light chain constant region and the heavy chain constant region are each from: a murine IgG1 antibody, a murine IgG4 antibody, or a mutant thereof; or a human IgG1 antibody, a human IgG4 antibody, or a mutant thereof.

According to an embodiment of the present disclosure, the light chain constant region includes a humanized or murine κ chain, λ chain, or a variant thereof.

According to an embodiment of the present disclosure, the antibody includes a heavy chain constant region having an amino acid sequence as set forth in SEQ ID NO: 27, SEQ ID NO:29, or SEQ ID NO:31; and/or a light chain constant region having an amino acid sequence as set forth in SEQ ID NO: 28, SEQ ID NO:30, or SEQ ID NO:32.

According to an embodiment of the present disclosure, the antibody includes a heavy chain constant region having an amino acid sequence as set forth in SEQ ID NO: 27; and a light chain constant region having an amino acid sequence as set forth in SEQ ID NO: 28.

According to an embodiment of the present disclosure, the antibody includes a heavy chain constant region having an amino acid sequence as set forth in SEQ ID NO: 29; and a light chain constant region having an amino acid sequence as set forth in SEQ ID NO: 30.

According to an embodiment of the present disclosure, the antibody includes a heavy chain constant region having an amino acid sequence as set forth in SEQ ID NO:31; and a light chain constant region having an amino acid sequence as set forth in SEQ ID NO:32.

According to an embodiment of the present disclosure, the antibody or antigen-binding fragment thereof includes a heavy chain having an amino acid sequence as set forth in any one of SEQ ID NO: 33, SEQ ID NO: 35, SEQ ID NO: 36, and SEQ ID NO: 38; and a light chain having an amino acid sequence as set forth in any one of SEQ ID NO: 34, SEQ ID NO: 37, and SEQ ID NO: 39.

According to an embodiment of the present disclosure, the antibody or antigen-binding fragment thereof includes:
a heavy chain having an amino acid sequence as set forth in SEQ ID NO: 33 and a light chain having an amino acid sequence as set forth in SEQ ID NO: 34;
a heavy chain having an amino acid sequence as set forth in SEQ ID NO: 35 and a light chain having an amino acid sequence as set forth in SEQ ID NO: 37;
a heavy chain having an amino acid sequence as set forth in SEQ ID NO: 36 and a light chain having an amino acid sequence as set forth in SEQ ID NO: 37; or
a heavy chain having an amino acid sequence as set forth in SEQ ID NO: 38 and a light chain having an amino acid sequence as set forth in SEQ ID NO: 39.

According to an embodiment of the present disclosure, the antibody or antigen-binding fragment thereof includes a monoclonal antibody or a polyclonal antibody.

According to an embodiment of the present disclosure, the monoclonal antibody includes at least one of Fv, single-chain antibody, Fab, single-domain antibody, and a minimal recognition unit.

According to an embodiment of the present disclosure, the antibody or antigen-binding fragment thereof is capable of binding to an amino acid sequence as set forth in SEQ ID NO: 40.

In a second aspect of the present disclosure, the present disclosure provides a nucleic acid molecule. The nucleic acid molecule encodes the antibody or antigen-binding fragment thereof according to the first aspect. According to some specific embodiments of the present disclosure, an antibody or antigen-binding fragment thereof encoded by the nucleic acid molecule can bind to the human CD94 protein and effectively treat or prevent a CD94-mediated disease.

According to an embodiment of the present disclosure, the above-mentioned nucleic acid molecule may further include at least one of the following additional technical features:

According to an embodiment of the present disclosure, the nucleic acid molecule is DNA.

It should be noted that, for the nucleic acids mentioned in the description and claims of the present disclosure those skilled in the art should understand that any one or two of complementary double strands are actually included. For convenience, although in most cases only one strand is given in the present description and the claims, the other strand complementary to the one strand is also disclosed actually. In addition, nucleotide sequences in the present disclosure include a DNA form or an RNA form. When one form is disclosed, it means that the other form is also disclosed.

In a third aspect of the present disclosure, the present disclosure provides an expression vector. The expression vector carries the nucleic acid molecule according to the second aspect. The expression vector may include an optional control sequence. The control sequence is operably linked with the nucleic acid molecule. The control sequence refers to one or more control sequences that can direct the expression of said nucleic acid molecule in the host. The expression vector provided in an embodiment of the present disclosure can efficiently and highly express the antibody or antigen-binding fragment thereof in suitable host cells.

The "operably linked" herein means that an exogenous gene is linked to a vector, enabling control components in the vector, such as a transcriptional control sequence and a translational control sequence, to exert their intended functions of regulating transcription and translation of the exogenous gene. When the nucleic acid molecule is linked to the vector, the nucleic acid molecule may be directly or indirectly connected with the control components on the vector, as long as these control components can control translation and expression of the nucleic acid molecule. These control components may be directly derived from the vector itself, and they may also be exogenous, that is, not derived from the vector itself. It may be understood by those skilled in the art that, the nucleic acid molecule used for coding the antibody or the antigen-binding fragment may be respectively and independently inserted into different vectors, and is commonly inserted into the same vector. Common vectors, for instance, may be plasmids, bacteriophages, etc., such as Plasmid-X.

In a fourth aspect of the present disclosure, the present disclosure provides a method for preparing the above-mentioned antibody or antigen-binding fragment thereof. The method includes: introducing the expression vector described above into cells; and culturing the cells under conditions suitable for protein expression and secretion, to obtain the antibody or antigen-binding fragment thereof. The method provided according to some specific embodiments of the present disclosure can effectively obtain a large quantity of the antibodies or antigen-binding fragments thereof in vitro.

According to some specific embodiments of the present disclosure, the method for preparing the above-mentioned antibody or antigen-binding fragment thereof may further include at least one of the following additional technical features.

According to some specific embodiments of the present disclosure, the cells are not particularly limited, and either prokaryotic cells or eukaryotic cells can be used.

According to some specific embodiments of the present disclosure, the cells are eukaryotic cells.

According to some specific embodiments of the present disclosure, the eukaryotic cells are mammalian cells. According to some specific embodiments of the present disclosure, when the cells are the eukaryotic cells, such as the mammalian cells, expression efficiency of the recombinant antibody is relatively high.

In a fifth aspect of the present disclosure, the present disclosure provides a recombinant cell. The recombinant cell carries the above-mentioned nucleic acid or expression vector, or is capable of expressing the aforementioned antibody or antigen-binding fragment thereof. The recombinant cell is obtained by transfection or transformation of the expression vector. According to some specific embodiments of the present disclosure, the recombinant cell can efficiently and and massively express the above-mentioned antibody or antigen-binding fragment thereof under appropriate conditions.

It should be noted that, the recombinant cell according to the present disclosure is not particularly limited, and it may be a prokaryotic cell, a eukaryotic cell, or a bacteriophage. The prokaryotic cell may be *Escherichia coli, Bacillus subtilis, Streptomyces,* or *Proteus mirabilis.* The eukaryotic cell includes: fungi such as *Pichia pastoris, Saccharomyces cerevisiae, Schizosaccharomyces,* and *Trichoderma;* insect cells such as grass armyworms; plant cells such as tobacco; and mammalian cells such as BHK cells, CHO cells, COS cells, and myeloma cells. In some embodiments, the recombinant cells according to the present disclosure are preferably the mammalian cells, including BHK cells, CHO cells, NSO cells, or COS cells and do not include animal germ cells, fertilized egg or embryonic stem cells.

It should be noted that, the "suitable conditions" in the description of the present disclosure refer to conditions suitable for the expression of the antibody or antigen-binding fragment thereof according to the present disclosure. Those skilled in the art can easily conceive that, the conditions suitable for the expression of the antibody or antigen-binding fragment thereof include, but not limited to, a suitable transformation or transfection method, a suitable transformation or transfection condition, a healthy host cell state, a suitable density of host cells, a suitable culture environment, and a suitable cell culture duration. The "suitable conditions" are not particularly limited. The most suitable conditions for the expression of the antibody or antigen-binding fragment thereof may be optimized by those skilled in the art based on specific laboratory environment.

In a sixth aspect of the present disclosure, the present disclosure provides an immunoconjugate. The immunoconjugate includes the above-mentioned antibody or antigen-binding fragment thereof, and a therapeutic agent. As mentioned above, the antibody or antigen-binding fragment thereof according to the embodiments of the present disclosure can effectively bind to the CD94 protein and can thus effectively treat or prevent a CD94-related disease. Therefore, the immunoconjugate includes the antibody or antigen-binding fragment thereof can also bind to the human CD94 protein. In this way, the immunoconjugate has good effects in preventing and/or treating a CD94-mediated disease.

In a seventh aspect of the present disclosure, the present disclosure provides a composition. The composition includes the above-mentioned antibody or antigen-binding fragment thereof, nucleic acid molecule, expression vector, or recombinant cell. As mentioned above, the antibody or antigen-binding fragment thereof according to some specific embodiments of the present disclosure can effectively bind to the huma CD94 protein and can thus effectively inhibit proliferation of tumor cells. Therefore, the composition including the above-mentioned substances can also effectively bind to the huma CD94 protein and can thus have good effects in preventing and/or treating a CD94-mediated disease. The type of the composition is not particularly limited. The composition may be a food composition or a pharmaceutical composition.

The composition according to the present disclosure can be combined with each other, or it can be administered in combination with one or more other therapeutic compounds, for example, in combination with a chemotherapeutic agent. Therefore, the composition may further include a chemotherapeutic agent. The antibody or the antigen-binding fragment thereof or the immunoconjugate according to the present disclosure may be in combination with a second therapeutic agent. Exemplary agents of the second therapeutic agent include, but not limited to: other reagents that inhibit CD94 activity, including other antibodies or antigen-binding fragments thereof, peptide inhibitors, small molecule antagonists, etc.; and/or agents interfering with upstream or downstream signal transduction of CD94.

It should be noted that, the composition includes combinations separated temporally or spatially, as long as they are capable of acting together to achieve objectives of the present disclosure. For example, components in the composition may be applied to a subject as a whole or separately. When the components contained in the composition are separately administered to the subject, the respective components may be simultaneously or sequentially administered to the subject.

In an eighth aspect of the present disclosure, the present disclosure provides a medicament. The medicament includes the above-mentioned antibody or antigen-binding fragment thereof, nucleic acid molecule, expression vector, recombinant cell, or composition. As mentioned above, the antibody or antigen-binding fragment thereof according to some specific embodiments of the present disclosure can effectively bind to the human CD94 protein. Therefore, the medicament including an effective amount of active ingredients of the antibody or antigen-binding fragment thereof or a series of substances thereof can also effectively bind to the human CD94 protein and has good effects in preventing and/or treating a CD94-mediated disease.

According to an embodiment of the present disclosure, the above-mentioned medicament may further include at least one of the following additional technical features.

According to an embodiment of the present disclosure, the medicament may further include a pharmaceutically acceptable carrier.

As used herein, the term "effective quantity" or "effective dose" refers to a quantity that may produce functions or activity to humans and/or animals and may be accepted by the humans and/or animals.

The effective dose of the antibody or antigen-binding fragment thereof according to the present disclosure may vary with the administration mode and the severity of the disease to be treated. The selection of the preferred effective dose may be determined by those skilled in the art based on various factors (such as through a clinical test). The factors include, but not limited to: pharmacokinetic parameters of active ingredients, for example, bioavailability, metabolism, and half-life period; severity of the disease to be treated in a patient; a weight of the patient; an immune status of the patient; and a route of administration. For example, based on the urgent requirements of treatment states, separated doses may be administrated several times per day, or the dose can be reduced proportionally.

As used herein, the "pharmaceutically acceptable" component is a substance that is suitable for use use in humans and/or mammals without excessive adverse side effects (such as, toxicity, irritation and allergy), that is, having a reasonable benefit/risk ratio. The term "pharmaceutically acceptable carrier" refers to a carrier used for therapeutic agent administration, including various excipients and diluents.

The medicament according to the present disclosure includes a safe and effective amount of the active component of the present disclosure and a pharmaceutically acceptable carrier. Such carriers include, but not limited to, saline, buffer solution, glucose, water, glycerin, ethanol and a combination thereof. Generally, a pharmaceutical preparation should be matched with an administration mode, which can be oral administration, intranasal administration, intradermal administration, subcutaneous administration, intramuscular administration or intravenous administration, or intraperitoneal administration. Dosage forms of the medicament according to the present disclosure include an injection, an oral preparation (tablets, capsules and oral liquid), a transdermal agents, and a sustained-release agent. For example, the medicament is prepared with normal saline or an aqueous solution containing glucose and other adjuvants by a conventional method. The medicament is suitable to be prepared under a sterile condition. The antibody or the antigen-binding fragment may be administered via intravenous infusion or injection or intramuscular or subcutaneous injection.

Of course, the anti-CD94 monoclonal antibody herein can also be formulated into a part of a kit or other diagnostic reagents as required.

In a ninth aspect of the present disclosure, the present disclosure provides a kit. The kit includes the above-mentioned antibody or antigen-binding fragment thereof, nucleic acid molecule, expression vector, or recombinant cell. As mentioned above, the antibody or antigen-binding fragment thereof according to some specific embodiments of the present disclosure can effectively bind to the human CD94 protein. Therefore, the kit including the antibody or antigen-binding fragment thereof can effectively performing qualitative or quantitative detection of the human CD94 protein. The kit provided by the present disclosure, may be used, for instance, in applications such as Western blotting, immunoprecipitation, or other assays that utilize the specific binding of huma CD94 and the antibody. Such a kit may include any or several of an antagonist, an anti-CD94 antibodies, or a medicament reference material; a protein purification columns; an immunoglobulin affinity purification buffering agent; an assay diluent for cells; an instruction or literature. The anti-CD94 antibody may be used for different types of diagnostic tests, for example, an in vitro or in vivo detection of various diseases or a presence of a medicament, a toxin, or other proteins. For example, the kit can be used to detect human CD94 protein in serum or blood samples from a subject, for the purpose of diagnosing related diseases, or for cientific researches. The related diseases may include a CD94 related disease, such as cancer. Certainly, the antibody or antigen-binding fragment thereof provided in the present disclosure may also be used for radioimmunoassay and radio immunotherapy of the above diseases. With respect to the above application scenarios, the binding molecule is also applicable, which is not described in detail herein.

The kit may further include, for example, a coating solution, which is conventional for CD94 detection.

In a tenth aspect of the present disclosure, the present disclosure provides a use of the above-mentioned antibody or antigen-binding fragment thereof, nucleic acid molecule, expression vector, recombinant cell, or composition in the preparation of a medicament for preventing and/or treating a CD94-mediated related disease. As mentioned above, the antibody or antigen-binding fragment thereof according to some specific embodiments of the present disclosure can effectively bind to the human CD94 protein. Therefore, the medicament including an effective amount of the antibody or antigen-binding fragment thereof or a series of substances thereof can also effectively bind to the human CD94 protein and can thus have good effects in preventing and/or treating a CD94-mediated disease.

According to an embodiment of the present disclosure, the above-mentioned use in the preparation of the medicament may further include at least one of the following additional technical features.

According to an embodiment of the present disclosure, the CD94-mediated related disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In an eleventh aspect of the present disclosure, the present disclosure provides a use of the above-mentioned antibody or antigen-binding fragment thereof, nucleic acid molecule, expression vector, or recombinant cell in the preparation of a kit for detecting CD94. As mentioned above, the antibody or antigen-binding fragment thereof according to some specific embodiments of the present disclosure can effectively bind to the human CD94 protein and block the binding of the CD94 protein to its receptor. Therefore, the antibody or antigen-binding fragment thereof can be used for preparing a kit for detecting the CD94 protein. The kit can effectively perform qualitative or quantitative detection of the human CD94 protein.

In a twelfth aspect of the present disclosure, the present disclosure provides a method for treating or preventing a CD94-mediated related disease. According to an embodiment of the present disclosure, the method includes administering to a subject at least one of: 1) the antibody or antigen-binding fragment thereof described above; 2) the nucleic acid molecule described above; 3) the expression vector described above; 4) the recombinant cell described above; 4) the composition described above; and 6) the medicament described above. As mentioned above, the antibody or antigen-binding fragment thereof can bind to the human CD94 protein and can thus effectively treat or prevent the CD94-mediated related disease, preferably an autoimmune disease or cancer. Therefore, the method according to the embodiment of the present disclosure can effectively treat or prevent the CD94-mediated related disease.

According to an embodiment of the present disclosure, the above-mentioned method for treating or preventing the disease may further include at least one of the following additional technical features.

According to an embodiment of the present disclosure, the CD94-mediated related disease includes a transplant rejection, an autoimmune disease, an infectious disease, and cancer. Those skilled in the art should understand that the CD94 antibodies are highly expressed on the surface of NK cells, and excessive activation of NK cells in sterile inflammation (a transplant rejection, an autoimmune disease) and bacterial inflammation (an infectious disease) can lead to tissue and organ damage. Therefore, the CD94 antibodies can be used as clearing antibodies to treat or prevent the above diseases.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of: lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In a thirteenth aspect of the present disclosure, the present disclosure provides a method for diagnosing a CD94-mediated related disease. According to an embodiment of the present disclosure, the method includes: detecting CD94 in a sample to be tested using at least one of: 1) the antibody or antigen-binding fragment thereof described above; 2) the nucleic acid molecule described above; 3) the expression vector described above; and 4) the recombinant cell described above; and determining, based on a detection result of CD94, a content of CD94 in the sample to be tested. The antibody or antigen-binding fragment thereof provided by the present disclosure, or antibodies or antigen-binding fragments expressed by the nucleic acid molecule, the expression vector, or the recombinant cell according to the present disclosure can effectively bind to the human CD94 protein, or antibodies or antigen-binding fragments expressed by the nucleic acid molecule, the expression vector, or the recombinant cell according to the present disclosure can effectively bind to CD94. Therefore, the method according to the present disclosure can effectively detect the content of CD94 in the sample to be tested that is from a subject, and thus can effectively diagnose related diseases caused by CD94.

According to an embodiment of the present disclosure, the above-mentioned method for diagnosing a disease may further include at least one of the following additional technical features.

According to an embodiment of the present disclosure, the content of CD94 in the sample to be tested being not lower than the lowest standard of suffering from such a disease is an indication that the sample to be tested is from a patient with the related disease caused by CD94. A value of the lowest standard may be determined by performing difference comparative analysis and validation on the contents of CD94 in samples to be tested that are from a large number of individuals suffering from the CD94-mediated disease and a large number of healthy individuals.

According to an embodiment of the present disclosure, the sample to be tested includes at least one of blood, saliva, sweat, tissue, cell, blood, serum, plasma, feces, and urine.

According to an embodiment of the present disclosure, the CD94-mediated related disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In a fourteenth aspect of the present disclosure, the present disclosure provides a method for staging a CD94-mediated related disease. According to an embodiment of the present disclosure, the method includes: detecting CD94 in a sample to be tested using at least one of: 1) the antibody or antigen-binding fragment thereof described above; 2) the nucleic acid molecule described above; 3) the expression vector described above; and 4) the recombinant cell described above; and determining, based on a detection result of the CD94, a content of CD94 in the sample to be tested. The antibody or antigen-binding fragment thereof provided by the present disclosure, or antibodies or antigen-binding fragments thereof expressed by the nucleic acid molecule, the expression vector and the recombinant cell according to the present disclosure can effectively bind to the huma CD94 protein. Therefore, the method according to the present disclosure can be use to effectively detect the content of CD94 in the sample to be tested from a subject, and can be further used to assess, based on the content of CD94, a stage of the related disease caused by CD94.

According to an embodiment of the present disclosure, the above-mentioned method for staging the disease may further include at least one of the following additional technical features.

According to an embodiment of the present disclosure, the content of CD94 in the sample to be tested being not lower than a standard level of patients suffering from the stage IV tumor is an indication that the sample to be tested is from a patient with a stage IV tumort; the content of CD94 in the sample to be tested being between a standard level of patients suffering from a stage IV tumor and a standard level of patients suffering from a stage III tumor is an indication that the sample to be tested is from a patient with the stage III tumor; the content of CD94 in the sample to be tested being between a standard level of patients suffering from a stage III tumor and a standard level of patients suffering from a stage II tumor is an indication that the sample to be tested is from a patient with a stage II tumor; and the content of CD94 in the sample to be tested being between a standard level of patients suffering from a stage I tumor and a standard level of patients suffering from a stage II tumor is an indication that the sample to be tested is from a patient with a stage I tumor. Those skilled in the art can understand that, the CD94 levels in patients suffering from the tumor stages I, II, III and IV varie with the types of the tumor; and the stages of the tumor can be determined by comparing the content of CD94 in the sample to be tested with the standard level of CD94 at a corresponding tumor stage, or by comparing the content of CD94 in the sample to be tested with a content of CD94 in samples from individuals or populations at a known disease stage. The values of the standard levels of the tumor stages I, II, III and IV can be determined by performing difference comparative analysis and validation on the contents of CD94 in samples to be tested of a large number of individuals suffering from the related diseases caused by CD94 and a large number of healthy individuals.

According to an embodiment of the present disclosure, the sample to be tested includes at least one of blood, saliva, sweat, tissue, cell, blood, serum, plasma, feces, and urine.

According to an embodiment of the present disclosure, the CD94-mediated related disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In a fifteenth aspect of the present disclosure, the present disclosure provides a method for assessing a prognosis of a CD94-mediated related disease. According to an embodiment of the present disclosure, the method includes: detecting CD94 in a sample to be tested using at least one of: 1) the antibody or antigen-binding fragment thereof described above; 2) the nucleic acid molecule described above; 3) the expression vector described above; and 4) the recombinant cell described above; and determining, based on a detection result of CD94, a content of the CD94 in the sample to be tested. As mentioned above, the content of CD94 has a significant influence on the cancers. After the individuals suffering from the related diseases are treated, the prognosis of the diseases may be effectively evaluated by monitoring the contents of CD94 in tissues or excreta (such as peripheral blood and urine) of the individuals, for instance, in a manner of comparing the contents of CD94 in the the subject before and after treatment, or comparing the contents of CD94 in the subject after treatment with the level of CD94 of the normal individuals or diseased individuals. The antibody or the antigen-binding fragment thereof provided by the present disclosure, or antibodies or antigen-binding fragments thereof expressed by the nucleic acid molecule, the expression vector and the recombinant cell according to the present disclosure can effectively bind to human CD94. Therefore, the method according to the present disclosure can effectively detect the content of CD94 in the sample to be tested of the tested individuals, and thus the method can further assess, based on the content of CD94, the prognosis of the related diseases caused by CD94.

According to an embodiment of the present disclosure, the above-mentioned method for assessing the disease prognosis may further include at least one of the following additional technical features.

According to an embodiment of the present disclosure, the sample to be tested is from a patient suffering from the CD94-mediated related disease before or after the treatment.

According to an embodiment of the present disclosure, the sample to be tested includes at least one of blood, saliva, sweat, tissue, cell, blood, serum, plasma, feces, and urine.

According to an embodiment of the present disclosure, a prognostic effect of the CD94-mediated related disease is determined based on the content of CD94 in the sample to be tested before or after treatment.

According to an embodiment of the present disclosure, a decrease in the content of CD94 in the sample to be tested of the patient suffering from the CD94-mediated disease after the treatment is an indication of a good prognosis for the patient.

According to an embodiment of the present disclosure, the CD94-mediated related disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In a sixteenth aspect of the present disclosure, the present disclosure provides a use of the aforementioned antibody or antigen-binding fragment thereof, nucleic acid molecule, expression vector, recombinant cell, composition, or medicament in the treatment or prevention of a CD94-mediated related disease. As mentioned above, the antibody or antigen-binding fragment thereof can effectively bind to human CD94 and can thus effectively treat or prevent a CD94-mediated related disease.

According to an embodiment of the present disclosure, the above-mentioned use may further include at least one of the following additional technical features.

According to an embodiment of the present disclosure, the CD94-mediated related disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

In a seventeenth aspect of the present disclosure, the present disclosure provides a use of the aforementioned antibody or antigen-binding fragment thereof, nucleic acid molecule, expression vector, or recombinant cell in the diagnosis, staging or assessment of a CD94-mediated disease. As mentioned above, the antibody or antigen-binding fragment thereof provided by the present disclosure, or antibodies or antigen-binding fragments thereof expressed by the nucleic acid molecule, the expression vector, and the recombinant cell according to the present disclosure can effectively bind to human CD94. Therefore, the method according to the present disclosure can effectively detect the content of CD94 in the sample to be tested that is from a subject and can thus effectively diagnose, stage, and assess the prognosis of a CD94-mediated disease.

According to an embodiment of the present disclosure, the above-mentioned use may further include at least one of the following additional technical features.

According to an embodiment of the present disclosure, the CD94-mediated disease includes a transplant rejection, an autoimmune disease, an infectious disease, and cancer.

According to an embodiment of the present disclosure, the autoimmune disease includes at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis.

According to an embodiment of the present disclosure, the cancer includes at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

The "subject" or "individual" involved in the present disclosure refer to a mammal, e.g., primate and/or rodent, particularly a human, a monkey, or a mouse.

### Beneficial effects of the present disclosure

1) So far, there have been no patents or research reports on the use of blocking CD94 antibodies in anti-cancer research.
2) The CD94 antibody obtained in the present disclosure has higher binding activity to the CD94 protein, and in the in vitro functional experiments, the activity of the CD94 antibody is superior to that of the NKG2A antibody in the clinical stage.

Additional aspects and advantages of the present disclosure will be provided at least in part in the following description, or will become apparent in part from the following description, or can be learned from the practice of the present disclosure.

### BRIEF DESCRIPTION OF THE DRAWINGS

The above and other aspects and advantages of embodiments of the present disclosure will become apparent and more readily appreciated from the following descriptions made with reference to the accompanying drawings.
FIG. 1 is a diagram showing the result of a human-mouse 15C10 chimeric antibody binding to 293 T-CD94/NKG2A cells according to an embodiment of the present disclosure.
FIG. 2 is a diagram showing the result of the blockade of HLA-E binding to 293T-CD94/NKG2A cells by a human-mouse 15C10 chimeric antibody according to an embodiment of the present disclosure.
FIG. 3 is a diagram showing the result of a human-mouse 15C10 chimeric antibody and a humanized 15C10 antibody binding to 293T-CD94/NKG2A cells according to an embodiment of the present disclosure.
FIG. 4 is a diagram showing the result of a humanized 15C10 antibody, and NKG2A antibodies huZ199 and huZ270 as controls binding to 293T-CD94/NKG2A cells according to an embodiment of the present disclosure.
FIG. 5 is a diagram showing the result of the blockade of HLA-E binding to 293T-CD94/NKG2A cells by a humanized 15C10 antibody according to an embodiment of the present disclosure.
FIG. 6 is an ELISA diagram showing the result of a humanized 15C10 antibody binding to a CD94/NKG2A-Fc heterodimer protein according to an embodiment of the present disclosure.
FIG. 7 is a diagram showing the result of a humanized 15C10 antibody promoting activation of Jurkat-NFAT-lucia-CD94/NKG2A T cells according to an embodiment of the present disclosure.
FIG. 8 is a diagram showing the result of a humanized 15C10 antibody promoting an anti-cancer effect in immune system-reconstituted mice according to an embodiment of the present disclosure.

### DETAILED DESCRIPTION

Embodiments of the present disclosure will be described in detail below. The embodiments described below are illustrative, are merely used for explaining the present disclosure, and cannot be understood as a limitation to the present disclosure.

It should be noted that, terms "first" and "second" are merely used for a descriptive purpose, but cannot be understood as an indication or implication of relative importance or an implied specification of the quantity of demonstrated technical features. Thus, features defined with the "first" and "second" may explicitly or implicitly include one or more features. Further, in the descriptions of the present disclosure, unless otherwise noted, the meaning of "more" refers to two or more than two.

The endpoints of the ranges and any values disclosed herein are not limited to the precise range or value, and such ranges or values should be understood to include values approximating such ranges or values. For numerical ranges, the endpoints of each range, the endpoints of each range, an endpoint value and an individual point value within each range, and individual point values can be combined with each other to obtain one or more new numerical ranges, and such numerical ranges are to be considered to be specifically disclosed herein.

For an easier understanding of the present disclosure, certain technical and scientific terms are specifically defined below. Unless obviously and clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

An antibody or antigen-binding fragment thereof according to the present disclosure is generally prepared by a biosynthesis method. According to the nucleotide sequences in the present disclosure, coded nucleic acids according to the present disclosure may be conveniently prepared by those skilled in the art using various known methods. For example, these methods include, but not limited to: PCR, DNA artificial synthesis, etc. A specific method may refer to J. Sambrook, *Molecular Cloning:* Laboratory Manual. As an implementation of the present disclosure, coded nucleic acid sequences of the present disclosure may be constructed by a method for synthesizingnucleotide sequences in segments and conducting overlap extension PCR. The antibody or the antigen fragment is numbered and defined by a Kabat numbering system.

The antibody of the present disclosure includes a murine antibody, a chimeric antibody, and a humanized antibody, and preferably, the humanized antibody.

The term "murine antibody" in the present disclosure refers to a monoclonal antibody against human CD94 prepared according to the common knowledge and skills in the art. During the preparation, a test subject is injected with an antigen, and then hybridomas that express antibodies having desired sequences or functional properties are isolated. In a preferred embodiment of the present disclosure, the murine CD94 antibody or antigen-binding fragment thereof may further includes a light chain constant region of a murine κ chain, a murine λ chain, or a variant thereof, or further includes a heavy chain constant region of murine IgG1, murine IgG2, murine IgG3, or a variant thereof.

The term "chimeric antibody" refers to an antibody formed by fusing the variable region of a murine antibody with the constant region of a human antibody, which can reduce the immune response induced by the murine antibody. The variable region genes are cloned from mouse hybridoma cells, and then the constant region genes of the human antibody are cloned as needed. The mouse variable region genes and human constant region genes are linked into chimeric genes and inserted into human vectors. Finally, the chimeric antibody molecules are expressed in eukaryotic expression systems or prokaryotic expression systems. In a preferred embodiment of the present disclosure, the antibody light chain of the CD94 chimeric antibody further includes a light chain Fc region of a humanized κ chain,λ chain, or a variant thereof. The antibody heavy chain of the CD94 chimeric antibody further includes a heavy chain constant region of humanized IgG1, humanized IgG2, humanized IgG3, humanized IgG4, or a variant thereof.

The term "humanized antibody", also known as CDR-grafted antibody, refers to an antibody produced by grafting mouse CDR sequences into a human antibody variable region framework, i.e., different types of human germline antibody framework sequences. Such framework sequences can be obtained from public DNA databases or published references that include germline antibody gene sequences. For example, germline DNA sequences of human heavy variable region genes and human light chain variable region genes can be found in the "VBase" human germline sequence database. In order to avoid a decrease in activity caused by the reduction immunogenicity, minimal reverse mutations or back mutations may be introduced into human antibody variable region framework sequences to maintain the activity.

As used herein, the "monoclonal antibody" refers to an antibody with a single antigen-binding site.

As used herein, the "polyclonal antibody" refers to an antibody with two or more different antigen-binding sites.

In the present disclosure, the term "mutant" or "variant" may refer to a molecule obtained by performing mutation of one or more nucleotides or amino acids on any natural or engineered molecule.

The term "complementary determining region" or "CDR" or "CDR sequence" refers to an amino acid sequence in the antibody which is responsible for antigen binding, and for instance, generally including: amino acid residues nearby 23-34(L1), 50-56(L2) and 89-97(L3) in a light chain variable region, and amino acid residues nearby 31-35B(H1), 50-65(H2) and 95-102(H3) in a heavy chain variable region (Kabat et al., Sequences of Proteins of Immunological Interest, 5th Ed. Public Health Service, National Institutes of Health, Bethesda, MD.(1991)); and/or amino acid residues from "hypervariable loop" (for instance, amino acid residues nearby 26-32(LI), 50-52(L2) and 91-96(L3) in the light chain variable region, and nearby 26-32(H1), 53-55(H2) and 96-101(H3) in the heavy chain variable region) (Chothia and Lesk J. Mol. Biol. 196: 901-917(1987)).

As used herein, when the term "identity" is used for describing amino acid sequences or nucleic acid sequences relative to reference sequences, and a percentage of the identical amino acids or nucleotides between two amino acid sequences or nucleic acid sequences is determined by a conventional method, for instance, see Ausubel, et al. eds. (1995), Current Protocols in Molecular Biology, Chapter 19 (Greene Publishing and Wiley-Interscience, 1995, New York); and ALIGN program (Dayhoff (1978), Atlas of Protein Sequence and Structure 5: Suppl.3 (National Biomedical Research Foundation, Washington, D.C.). Various algorithms are applicable to sequence alignment and sequence identity determination, including: a homology alignment algorithm of Needleman et al. (1970) J.Mol.Biol.48: 443; a local homology algorithm of Smith et al. (1981) Adv.Appl.Math.2: 482; a similarity search method of Pearson et al. (1988) Proc.Natl.Acad.Sci.85: 2444; a Smith-Waterman algorithm (Meth.Mol.Biol.70: 173-187(1997)); and BLASTP, BLASTN and BLASTX algorithms (see Altschul et al. (1990) J.Mol.Biol.215: 403-410). Computer programs employing these algorithms are also available, and they include, but not limited to: ALIGN or Megalign (DNASTAR) software, or WU-BLAST-2 (Altschul et al., Meth. Enzym., 266: 460-480(1996)); or GAP, BESTFIT, BLAST Altschul etc., supra, FASTA and TFASTA, available in Genetics Computing Group (GCG) packet, Version 8, Madison, Wisconsin, USA; and CLUSTAL in a PC/Gene program provided by Intelligenetics, Mountain View, California.

Without substantially affecting antibody activity (remaining at least 95% of activity), substitution, addition and/or deletion of one or more amino acids (such as, 1, 2, 3, 4, 5, 6, 7, 8, 9, or 10 or more) may be conducted by those skilled in the art on the sequences in the present disclosure, to obtain variants of sequences of the antibody or functional fragments thereof. These variants are regarded as being included in the protection scope of the present disclosure. For example, amino acids with similar properties are substituted in a variable region. The variant sequences in the present disclosure may have at least 80%, 85%, 90%, 95%, 96%, 97%, 98% or 99% identity (or homology) to the reference sequences. The sequence identity in the present disclosure may be measured by sequence analysis software, such as a computer program BLAST using a default parameter, particularly BLASTP or TBLASTN. The amino acid sequences described in the present disclosure are all shown from the N-terminus to the C-terminus.

As mentioned above, the monoclonal antibody according to the present disclosure may be a full-length antibody or may only include functional fragments thereof (such as, Fab, F(ab')2 or scFv fragments), or may be modified to affect the function. The present disclosure includes an anti-CD94 antibody with a modified glycosylation patterns. In some applications, performing modification to remove unexpected glycosylation sites may be useful, or eliminate the presence of fucose moieties on the oligosaccharide chains, for instance, to enhance antibody-dependent cellular cytotoxicity (ADCC) function. In some other applications, galactosylation modification may be performed to change complement-dependent cytotoxcity (CDC).

As used herein, the "full-length antibody" is of a tetrapeptide chain structure formed by linking two identical light chains and two identical heavy chains through interchain disulfide bonds, such as immunoglobulin G (IgG), immunoglobulin A (IgA), immunoglobulin M (IgM), immunoglobulin D (IgD) or immunoglobulin E (IgE). The immunoglobulin of the same class may also be divided into different subclasses according to amino acid compositions, such as IgG1, IgG2, IgG3, and IgG4. The light chain of the immunoglobulin is divided into κ chain or λ chain according to different constant regions.

As used herein, the term "functional fragment" particularly refers to a fragment of an antibody, such as a CDR transplant antibody, Fab, Fab', (Fab')2, Fv or scFv, a nanobody, or any fragment, which can prolong half-life through chemical modification or by being doped into lipidosome. The chemical modification, for instance, refers to addition of poly(alkylene) glycol, such as polyethylene glycol ("pegylation, PEGylation") (called pegylated fragments of Fv-PEG, scFv-PEG, Fab-PEG, F(ab')2-PEG or Fab'-PEG) ("PEG" is the polyethylene glycol); and the fragment has CD94 binding activity. Preferably, the functional fragment will consist of or contain a partial sequence of the heavy or light chain variable region of its source antibody. The partial sequence is enough to remain binding specificity identical to the source antibody and sufficient affinity, for CD94, preferably at least equal to 1/100 of the affinity of the source antibody, at least equal to 1/10 in a more preferred mode. The functional fragment will include at least 3 amino acids, preferably 5, 10, 15, 25, 50, and 100 consecutive amino acids in the sequence of the source antibody.

In the present disclosure, unless contrary statement is given, the used term "antigen-binding fragment" generally refers to an antigen-binding antibody fragment, and it may include a portion of a complete antibody, generally an antigen-binding region or a variable region, for example, including a CDR transplant antibody, Fab, Fab', F(ab')2, Fv or scFv, nanobody, etc.

As used herein, the term "CDR-grafted antibody" refers to the CDR of a monoclonal antibody of one species is grafted to a variable region of an antibody of another species. For example, the CDR of a murine monoclonal antibody may be grafted to a variable region of a humanized antibody to replace the CDR of the humanized antibody, so that the humanized antibody obtains antigen-binding specificity of the murine monoclonal antibody, thereby decreasing heterology of the antibody.

As used herein, the term "Fab antibody" or "Fab" generally refers to an antibody including only Fab molecule, and is composed of VH and CH1 of a heavy chain and a complete light chain, with the light chain and the heavy chain linked through a disulfide bond.

As used herein, the term "nanobody" (a single domain antibody or a VHH antibody), which is originally described as an antigen-binding immunoglobulin (variable) domain of a "heavy chain antibody" (that is, "an antibody lacking a light chain") (Hamers-Casterman C, AtarhouchT, Muyldermans S, Robinson G, Hamers C, Songa EB, Bendahman N, Hamers R.: "Naturally occurring antibodies devoid of light chains"; Nature 363,446-448(1993)), only includes a heavy chain variable region (VH) and conventional CH2 and CH3 regions, and is specifically bound to an antigen through the heavy chain variable region.

As used herein, the term "Fv antibody" generally refers to an antibody formed by linking a light chain variable region (VL) and a heavy chain variable region (VH) through noncovalent bonds, and is the minimum functional fragment of an antibody molecule that remains a complete antigen binding site.

As used herein, the term "single chain antibody" or "scFv" is a fragment formed by linking a heavy chain variable region and a light chain variable region of the antibody through a short chain polypeptide.

The amino acid or nucleic acid sequences involved in the present disclosure are specifically listed in Table 1.

**[Table 1]**

| SEQ ID NO: | Sequence | Description |
|---|---|---|
| 1 | GFNIKDTY | Heavy chain variable region CDR1 (HCDR1) of murine antibody |
| 2 | IDPANVNT | Heavy chain variable region CDR2 (HCDR2) of murine antibody |
| 3 | VGGRGHTWFAY | Heavy chain variable region CDR3 (HCDR3) of murine antibody |
| 4 | QDITNY | Light chain variable region CDR1 (LCDR1) of murine antibody |
| 5 | YTS | Light chain variable region CDR2 (LCDR2) of murine antibody |
| 6 | QQGNTLPYT | Light chain variable region CDR3 (LCDR3) of murine antibody |
| 7 | EVQLQQSGAELVMPGASVKLSCTAS | Heavy chain variable region FR1 (mHFR1) of murine antibody |
| 8 | MHWVMQRPEQGLEWIGR | Heavy chain variable region FR2 (mHFR2) of murine antibody |
| 9 | NYDPRFQGKATITADTSSNTAYLQFSSLTSEDTAVYYC | Murine antibody heavy chain variable region FR3 (mHFR3) |
| 10 | WGQGTLVTVSA | Heavy chain variable region FR4 (mHFR4) of murine antibody |
| 11 | DIQMTQTTSSLSASLGDRVTISCRAS | Light chain variable region FR1 (mLFR1) of murine antibody |
| 12 | LNWYQQKPDGTVKLLIY | Light chain variable region FR2 (mLFR2) of murine antibody |
| 13 | RLHSGVPSRFSGSGSGTDYSLTISNLEQEDIATYFC | Light chain variable region FR3 (mLFR3) of murine antibody |
| 14 | FGGGTKLEIK | Light chain variable region FR4 (mLFR4) of murine antibody |
| 15 | QVQLVQSGAEVKKPGASVKVSCKAS | Heavy chain variable region FR1 (hHFR1) of humanized antibody h15C10-hIgG4 |
| 16 | MHWVRQAPGQGLEWIGR | Heavy chain variable region FR2 (hHFR2) of humanized antibody h15C10-hIgG4 |
| 17 | NYDQKFQGRVTMTRDTSTSTVYMELSSLRSEDTAVYYC | Heavy chain variable region FR3 (hHFR3) of humanized antibody h15C10-hIgG4 |
| 18 | WGQGTTVTVSS | Heavy chain variable region FR4 (hHFR4) of humanized antibody h15C10-hIgG4 |
| 19 | DIQMTQSPSSLSASVGDRVTITCRAS | Light chain variable region FR1 (LFR1) of humanized antibody h15C10-hIgG4 |
| 20 | LNWYQQKPGKAPKLLIY | Light chain variable region FR2 (LFR2) of humanized antibody h15C10-hIgG4 |
| 21 | RLHSGVPSRFSGSGSGTDYTLTISSLQPEDFATYYC | Light chain variable region FR3 (LFR3) of humanized antibody h15C10-hIgG4 |
| 22 | FGQGTKLEIK | Light chain variable region FR4 (hLFR4) of humanized antibody h15C10-hIgG4 |
| 23 | EVQLQQSGAELVMPGASVKLSCTASGFNIKDTYMHWVMQRPE QGLEWIGRIDPANVNTNYDPRFQGKATITADTSSNTAYLQFSSLT SEDTAVYYCVGGRGHTWFAYWGQGTLVTVSA | Heavy chain variable region of murine antibody/human-mouse chimeric antibody 15C10-hIgG1mut or 15C10-hIgG4 |
| 24 | DIQMTQTTSSLSASLGDRVTISCRASQDITNYLNWYQQKPDGTV KLLIYYTSRLHSGVPSRFSGSGSGTDYSLTISNLEQEDIATYFCQQ GNTLPYTFGGGTKLEIK | Light chain variable region of murine antibody/human-mouse chimeric antibody 15C10-hIgG1mut or 15C10-hIgG4 |
| 25 | QVQLVQSGAEVKKPGASVKVSCKASGFNIKDTYMHWVRQAPG QGLEWIGRIDPANVNTNYDQKFQGRVTMTRDTSTSTVYMELSS LRSEDTAVYYCVGGRGHTWFAYWGQGTTVTVSS | Heavy chain variable region of humanized antibody h15C10-hIgG4 |
| 26 | DIQMTQSPSSLSASVGDRVTITCRASQDITNYLNWYQQKPGKAP KLLIYYTSRLHSGVPSRFSGSGSGTDYTLTISSLQPEDFATYYCQQ GNTLPYTFGQGTKLEIK | Light chain variable region of humanized antibody h15C10-hIgG4 |
| 27 | AKTTAPSVYPLAPVCGDTTGSSVTLGCLVKGYFPEPVTLTWNSG SLSSGVHTFPAVLQSDLYTLSSSVTVTSSTWPSQSITCNVAHPASS TKVDKKIEPRGPTIKPCPPCKCPAPNLLGGPSVFIFPPKIKDVLMI SLSPIVTCVVVDVSEDDPDVQISWFVNNVEVHTAQTQTHREDY NSTLRVVSALPIQHQDWMSGKEFKCKVNNKDLPAPIERTISKPK GSVRAPQVYVLPPPEEEMTKKQVTLTCMVTDFMPEDIYVEWTN NGKTELNYKNTEPVLDSDGSYFMYSKLRVEKKNWVERNSYSC SVVHEGLHNHHTTKSFSRTPGK | Heavy chain constant region of murine antibody |
| 28 | RADAAPTVSIFPPSSEQLTSGGASVVCFLNNFYPKDINVKWKIDG SERQNGVLNSWTDQDSKDSTYSMSSTLTLTKDEYERHNSYTCE ATHKTSTSPIVKSFNRNEC | Light chain constant region of murine antibody |
| 29 | ASTKGPSVFPLAPSSKSTSGGTAALGCLVKDYFPEPVTVSWNSG ALTSGVHTFPAVLQSSGLYSLSSVVTVPSSSLGTQTYICNVNHKP SNTKVDKKVEPKSCDKTHTCPPCPAPELLGGPSVFLFPPKPKDTL MISRTPEVTCVVVAVSHEDPEVKFNWYVDGVEVHNAKTKPREE QYASTYRVVSVLTVLHQDWLNGKEYKCKVSNKALPAPIEKTISK AKGQPREPQVYTLPPSREEMTKNQVSLTCLVKGFYPSDIAVEWE SNGQPENNYKTTPPVLDSDGSFFLYSKLTVDKSRWQQGNVFSCS VMHEALHNHYTQKSLSLSPGK | Heavy chain constant region of human-mouse chimeric antibody 15C10-hIgGmut |
| 30 | | Light chain constant region of human-mouse chimeric antibody 15C10-hIgG1mut/15C10-hIgG4 |
| 31 | ASTKGPSVFPLAPCSRSTSESTAALGCLVKDYFPEPVTVSWNSG ALTSGVHTFPAVLQSSGLYSLSSVVTVPSSSLGTKTYTCNVDHKP SNTKVDKRVESKYGPPCPPCPAPEFLGGPSVFLFPPKPKDTLMIS RTPEVTCVVVDVSQEDPEVQFNWYVDGVEVHNAKTKPREEQF NSTYRVVSVLTVLHQDWLNGKEYKCKVSNKGLPSSIEKTISKAK GQPREPQVYTLPPSQEEMTKNQVSLTCLVKGFYPSDIAVEWESN GQPENNYKTTPPVLDSDGSFFLYSRLTVDKSRWQEGNVFSCSV MHEALHNHYTQKSLSLSLGK | Heavy chain constant region of humanized antibody h15C10-hIgG4/human-mouse chimeric antibody 15C10-hIgG4 |
| 32 | | Light chain constant region of humanized antibody h15C10-hIgG4 |
| 33 | EVQLQQSGAELVMPGASVKLSCTASGFNIKDTYMHWVMQRPE QGLEWIGRIDPANVNTNYDPRFQGKATITADTSSNTAYLQFSSLT SEDTAVYYCVGGRGHTWFAYWGQGTLVTVSAAKTTAPSVYPLA PVCGDTTGSSVTLGCLVKGYFPEPVTLTWNSGSLSSGVHTFPAV LQSDLYTLSSSVTVTSSTWPSQSITCNVAHPASSTKVDKKIEPRGP TIKPCPPCKCPAPNLLGGPSVFIFPPKIKDVLMISLSPIVTCVVVD VSEDDPDVQISWFVNNVEVHTAQTQTHREDYNSTLRVVSALPIQ HQDWMSGKEFKCKVNNKDLPAPIERTISKPKGSVRAPQVYVLP PPEEEMTKKQVTLTCMVTDFMPEDIYVEWTNNGKTELNYKNTE PVLDSDGSYFMYSKLRVEKKNWVERNSYSCSVVHEGLHNHHT TKSFSRTPGK | Heavy chain of anti-CD94 murine antibody |
| 34 | DIQMTQTTSSLSASLGDRVTISCRASQDITNYLNWYQQKPDGTV KLLIYYTSRLHSGVPSRFSGSGSGTDYSLTISNLEQEDIATYFCQQ GNTLPYTFGGGTKLEIKRADAAPTVSIFPPSSEQLTSGGASVVCF LNNFYPKDINVKWKIDGSERQNGVLNSWTDQDSKDSTYSMSST LTLTKDEYERHNSYTCEATHKTSTSPIVKSFNRNEC | Light chain of anti-CD94 murine antibody |
| 35 | EVQLQQSGAELVMPGASVKLSCTASGFNIKDTYMHWVMQRPE QGLEWIGRIDPANVNTNYDPRFQGKATITADTSSNTAYLQFSSLT SEDTAVYYCVGGRGHTWFAYWGQGTLVTVSAASTKGPSVFPLA | Heavy chain of human-mouse chimeric antibody 15C10-hIgG1mut |
| | PSSKSTSGGTAALGCLVKDYFPEPVTVSWNSGALTSGVHTFPAV LQSSGLYSLSSVVTVPSSSLGTQTYICNVNHKPSNTKVDKKVEP KSCDKTHTCPPCPAPELLGGPSVFLFPPKPKDTLMISRTPEVTCV VVAVSHEDPEVKFNWYVDGVEVHNAKTKPREEQYASTYRVVS VLTVLHQDWLNGKEYKCKVSNKALPAPIEKTISKAKGQPREPQ VYTLPPSREEMTKNQVSLTCLVKGFYPSDIAVEWESNGQPENNY KTTPPVLDSDGSFFLYSKLTVDKSRWQQGNVFSCSVMHEALHN HYTQKSLSLSPGK | |
| 36 | EVQLQQSGAELVMPGASVKLSCTASGFNIKDTYMHWVMQRPE QGLEWIGRIDPANVNTNYDPRFQGKATITADTSSNTAYLQFSSLT SEDTAVYYCVGGRGHTWFAYWGQGTLVTVSAASTKGPSVFPLA PCSRSTSESTAALGCLVKDYFPEPVTVSWNSGALTSGVHTFPAVL QSSGLYSLSSVVTVPSSSLGTKTYTCNVDHKPSNTKVDKRVESK YGPPCPPCPAPEFLGGPSVFLFPPKPKDTLMISRTPEVTCVVVDV SQEDPEVQFNWYVDGVEVHNAKTKPREEQFNSTYRVVSVLTVL HQDWLNGKEYKCKVSNKGLPSSIEKTISKAKGQPREPQVYTLPP SQEEMTKNQVSLTCLVKGFYPSDIAVEWESNGQPENNYKTTPPV LDSDGSFFLYSRLTVDKSRWQEGNVFSCSVMHEALHNHYTQKS LSLSLGK | Heavy chain of human-mouse chimeric antibody 15C10-hIgG4 |
| 37 | DIQMTQTTSSLSASLGDRVTISCRASQDITNYLNWYQQKPDGTV KLLIYYTSRLHSGVPSRFSGSGSGTDYSLTISNLEQEDIATYFCQQ GNTLPYTFGGGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC | Light chain of human-mouse chimeric antibody 15C10-hIgG1mut/15C10-hIgG4 |
| 38 | QVQLVQSGAEVKKPGASVKVSCKASGFNIKDTYMHWVRQAPG QGLEWIGRIDPANVNTNYDQKFQGRVTMTRDTSTSTVYMELSS LRSEDTAVYYCVGGRGHTWFAYWGQGTTVTVSSASTKGPSVFP LAPCSRSTSESTAALGCLVKDYFPEPVTVSWNSGALTSGVHTFPA VLQSSGLYSLSSVVTVPSSSLGTKTYTCNVDHKPSNTKVDKRVE SKYGPPCPPCPAPEFLGGPSVFLFPPKPKDTLMISRTPEVTCVVV DVSQEDPEVQFNWYVDGVEVHNAKTKPREEQFNSTYRVVSVLT VLHQDWLNGKEYKCKVSNKGLPSSIEKTISKAKGQPREPQVYT LPPSQEEMTKNQVSLTCLVKGFYPSDIAVEWESNGQPENNYKTT PPVLDSDGSFFLYSRLTVDKSRWQEGNVFSCSVMHEALHNHYT QKSLSLSLGK | Heavy chain of humanized antibody h15C10-hIgG4 |
| 39 | DIQMTQSPSSLSASVGDRVTITCRASQDITNYLNWYQQKPGKAP KLLIYYTSRLHSGVPSRFSGSGSGTDYTLTISSLQPEDFATYYCQQ GNTLPYTFGQGTKLEIKRTVAAPSVFIFPPSDEQLKSGTASVVCL LNNFYPREAKVQWKVDNALQSGNSQESVTEQDSKDSTYSLSST LTLSKADYEKHKVYACEVTHQGLSSPVTKSFNRGEC | Light chain of humanized antibody h15C10-hIgG4 |
| 40 | MAVFKTTLWRLISGTLGIICLSLMATLGILLKNSFTKLSIEPAFTPG PNIELQKDSDCCSCQEKWVGYRCNCYFISSEQKTWNESRHLCA SQKSSLLQLQNTDELDFMSSSQQFYWIGLSYSEEHTAWLWENGS ALSQYLFPSFETFNTKNCIAYNPNGNALDESCEDKNRYICKQQLI | Amino acid sequence of CD94 protein |
| 41 | ATGGCAGTCTTTAAGACCACACTGTGGAGGCTGATAAGCGGC ACCCTGGGGATCATATGCCTGTCACTTATGGCTACTCTCGGCAT CTTGCTGAAGAACAGTTTCACCAAGCTGAGCATCGAGCCAGC TTTCACCCCCGGTCCCAACATCGAGCTCCAGAAGGATTCCGA CTGTTGCTCCTGCCAGGAAAAGTGGGTGGGCTACAGGTGTAA CTGCTATTTCATATCCTCAGAGCAGAAGACATGGAACGAATCC CGACATCTGTGCGCCAGCCAGAAGTCAAGCTTGCTGCAGCTC CAGAACACCGATGAGCTTGACTTTATGAGCTCCTCTCAGCAGT TCTATTGGATTGGGTTGAGCTATTCTGAAGAGCATACTGCATG GTTGTGGGAAAACGGCTCAGCCCTCTCACAATATCTTTTCCCT TCATTTGAAACCTTTAACACCAAGAATTGCATTGCCTATAACC CAAATGGCAACGCACTGGATGAGTCCTGCGAAGATAAAAACC GATACATCTGTAAGCAGCA GCTTATC | Nucleotide sequence encoding CD94 protein |
| 42 | MDNQGVIYSDLNLPPNPKRQQRKPKGNKNSILATEQEITYAELN LQKASQDFQGNDKTYHCKDLPSAPEKLIVGILGIICLILMASVVT IVVIPSTLIQRHNNSSLNTRTQKARHCGHCPEEWITYSNSCYYIG KERRTWEESLLACTSKNSSLLSIDNEEEMKFLSIISPSSWIGVFRN SSHHPWVTMNGLAFKHEIKDSDNAELNCAVLQVNRLKSAQCGS SIIYHCKHKL | Amino acid sequence of NKG2A protein |
| 43 | ATGGACAACCAGGGCGTGATCTATTCTGACTTGAACCTGCCTC CCAACCCAAAACGCCAGCAGAGAAAGCCAAAGGGGAATAAG AACAGTATCCTCGCCACAGAGCAGGAGATTACTTACGCCGAG CTCAACCTGCAGAAGGCATCTCAGGATTTTCAAGGAAACGAT AAAACCTACCACTGCAAAGACTTGCCTTCCGCTCCTGAAAAG CTGATCGTCGGCATTCTCGGTATCATCTGCCTCATCCTTATGGC CTCCGTGGTGACAATTGTCGTCATTCCATCCACACTGATCCAA CGACACAATAATTCTTCTCTGAATACCAGGACACAGAAGGCC AGACATTGCGGCCATTGTCCTGAGGAATGGATCACCTATTCCA ACTCTTGTTATTATATTGGGAAGGAGCGCAGAACTTGGGAAG AATCTCTGCTCGCTTGCACATCTAAGAACAGTTCTCTCCTGTC CATCGACAATGAAGAAGAGATGAAGTTTCTGAGCATCATTTCT CCCTCCTCCTGGATCGGGGTGTTTCGTAACTCAAGTCATCATC CTTGGGTGACTATGAATGGATTGGCCTTTAAGCACGAAATCAA GGATTCCGATAATGCTGAGCTTAACTGTGCCGTCCTGCAAGTC AATAGGCTGAAGTCCGCTCAGTGCGGCTCCTCTATTATATACC ACTGCAAA CATAAGCTG | Nucleotide sequence encoding NKG2A protein |
| 44 | QVQLVQSGAEVKKPGASVKVSCKASGYTFTSYWMNWVRQAP GQGLEWMGRIDPYDSETHYAQKLQGRVTMTTDTSTSTAYMELR SLRSDDTAVYYCARGGYDFDVGTLYWFFDVWGQGTTVTVSAS TKGPSVFPLAPSSKSTSGGTAALGCLVKDYFPEPVTVSWNSGAL TSGVHTFPAVLQSSGLYSLSSVVTVPSSSLGTQTYICNVNHKPSN TKVDKKVEPKSCDKTHTCPPCPAPELLGGPSVFLFPPKPKDTLMI SRTPEVTCVVVAVSHEDPEVKFNWYVDGVEVHNAKTKPREEQY ASTYRVVSVLTVLHQDWLNGKEYKCKVSNKALPAPIEKTISKAK GQPREPQVYTLPPSREEMTKNQVSLTCLVKGFYPSDIAVEWESN GQPENNYKTTPPVLDSDGSFFLYSKLTVDKSRWQQGNVFSCSV MHEALHNHYTQKSLSLSPGK | Heavy chain of huZ270-hIgG1mut antibody |
| 45 | DIQMTQSPSSLSASVGDRVTITCRASENIYSYLAWYQQKPGKAP KLLIYNAKTLAEGVPSRFSGSGSGTDFTLTISSLQPEDFATYYCQ HHYGTPRTFGGGTKVEIKRTVAAPSVFIFPPSDEQLKSGTASVVC LLNNFYPREAKVQWKVDNALQSGNSQESVTEQDSKDSTYSLSS TLTLSKADYEKHKVYACEVTHQGLSSPVTKSFNRGEC | Light chain of huZ270-hIgG1mut antibody |
| 46 | EVQLVESGGGLVKPGGSLRLSCAASGFTFSSYAMSWVRQAPGK GLEWVSEISSGGSYTYYADSVKGRFTISRDNAKNSLYLQMNSLR AEDTAVYYCARHGDYPRFFDVWGQGTTVTVSSASTKGPSVFPL APCSRSTSESTAALGCLVKDYFPEPVTVSWNSGALTSGVHTFPAV LQSSGLYSLSSVVTVPSSSLGTKTYTCNVDHKPSNTKVDKRVES KYGPPCPPCPAPEFLGGPSVFLFPPKPKDTLMISRTPEVTCVVVD VSQEDPEVQFNWYVDGVEVHNAKTKPREEQFNSTYRVVSVLTV LHQDWLNGKEYKCKVSNKGLPSSIEKTISKAKGQPREPQVYTLP PSQEEMTKNQVSLTCLVKGFYPSDIAVEWESNGQPENNYKTTPP VLDSDGSFFLYSRLTVDKSRWQEGNVFSCSVMHEALHNHYTQK SLSLSLGK | Heavy chain of huZ199- hIgG4 antibody |
| 47 | EIVLTQSPATLSLSPGERATLSCSASSSVSSYIYWYQQKPGQAPRL LIYLTSNLASGIPARFSGSGSGTDFTLTISSLEPEDFAVYYCQQWS GNPYTFGQGTKLEIKRTVAAPSVFIFPPSDEQLKSGTASVVCLLN NFYPREAKVQWKVDNALQSGNSQESVTEQDSKDSTYSLSSTLT LSKADYEKHKVYACEVTHQGLSSPVTKSFNRGEC | Light chain of huZ199-hIgG4 antibody |
| 48 | QVQLVQSGAEVKKPGASVKVSCKASGYTFTSYWMNWVRQAP GQGLEWMGRIDPYDSETHYAQKLQGRVTMTTDTSTSTAYMELR | Heavy chain of huZ270-hIgG4 antibody |
| | SLRSDDTAVYYCARGGYDFDVGTLYWFFDVWGQGTTVTVSAS TKGPSVFPLAPCSRSTSESTAALGCLVKDYFPEPVTVSWNSGALT SGVHTFPAVLQSSGLYSLSSVVTVPSSSLGTKTYTCNVDHKPSNT KVDKRVESKYGPPCPPCPAPEFLGGPSVFLFPPKPKDTLMISRTPE VTCVVVDVSQEDPEVQFNWYVDGVEVHNAKTKPREEQFNSTY RVVSVLTVLHQDWLNGKEYKCKVSNKGLPSSIEKTISKAKGQPR EPQVYTLPPSQEEMTKNQVSLTCLVKGFYPSDIAVEWESNGQPE NNYKTTPPVLDSDGSFFLYSRLTVDKSRWQEGNVFSCSVMHEA LHNHYTQKSLSLSLGK | |
| 49 | DIQMTQSPSSLSASVGDRVTITCRASENIYSYLAWYQQKPGKAP KLLIYNAKTLAEGVPSRFSGSGSGTDFTLTISSLQPEDFATYYCQ HHYGTPRTFGGGTKVEIKRTVAAPSVFIFPPSDEQLKSGTASVVC LLNNFYPREAKVQWKVDNALQSGNSQESVTEQDSKDSTYSLSS TLTLSKADYEKHKVYACEVTHQGLSSPVTKSFNRGEC | Light chain of huZ270-hIgG4 antibody |
| 50 | PKSCDKTHTCPPCPAPELLGGPSVFLFPPKPKDTLMISRTPEVTC VVVAVSHEDPEVKFNWYVDGVEVHNAKTKPREEQYASTYRVV SVLTVLHQDWLNGKEYKCKVSNKALPAPIEKTISKAKGQPREPQ VYTLPPSREEMTKNQVSLWCLVKGFYPSDIAVEWESNGQPENN YKTTPPVLDSDGSFFLYSKLTVDKSRWQQGNVFSCSVMHEALH NHYTQKSLSLSPGKKNSFTKLSIEPAFTPGPNIELQKDSDCCSCQ EKWVGYRCNCYFISSEQKTWNESRHLCASQKSSLLQLQNTDEL DFMSSSAQFYWIGLSYSEEHTAWLWENGSALSQYLFPSFETFNT KNCIAYNPNGNALDESCEDKNRYICKQQLI | CD94-Femutknob amino acid sequence |
| 51 | PKSCDKTHTCPPCPAPELLGGPSVFLFPPKPKDTLMISRTPEVTC VVVAVSHEDPEVKFNWYVDGVEVHNAKTKPREEQYASTYRVV SVLTVLHQDWLNGKEYKCKVSNKALPAPIEKTISKAKGQPREPQ VYTLPPSREEMTKNQVSLSCAVKGFYPSDIAVEWESNGQPENNY KTTPPVLDSDGSFFLVSKLTVDKSRWQQGNVFSCSVMHEALHN HYTQKSLSLSPGKPSTLIQRHNNSSLNTRTQKARHCGHCPEEWI TYSNSCYYIGKERRTWEESLLACTSKNSSLLSIDNEEEMKFLSIIS PSSWIGVFRNSSHHPWVTMNGLAFKHEIKDSDNAELNCAVLQV NRLKSAQCGSSIIYHCKHKL | NKG2A-Fcmuthole amino acid sequence |
| 52 | MVDGTLLLLLSEALALTQTWAGSHSLKYFHTSVSRPGRGEPRFI SVGYVDDTQFVRFDNDAASPRMVPRAPWMEQEGSEYWDRETR SARDTAQIFRVNLRTLRGYYNQSEAGSHTLQWMHGCELGPDGR FLRGYEQFAYDGKDYLTLNEDLRSWTAVDTAAQISEQKSNDASE AEHQRAYLEDTCVEWLHKYLEKGKETLLHLEPPKTHVTHHPIS DHEATLRCWALGFYPAEITLTWQQDGEGHTQDTELVETRPAGD GTFQKWAAVVVPSGEEQRYTCHVQHEGLPEPVTLRWKPASQPTI PIVGIIAGLVLLGSVVSGAVVAAVIWRKKSSGGKGGSYSKAEWS DSAQGSESHSL | Amino acid sequence of HLA-E protein |
| 53 | ATGGTGGATGGAACACTGCTCCTGTTGCTTTCTGAGGCTCTGG CTTTGACTCAGACTTGGGCCGGGAGCCATAGCCTGAAATATTT CCACACAAGTGTTTCTAGGCCTGGAAGAGGCGAACCAAGGTT CATCAGTGTCGGCTATGTGGACGACACTCAGTTCGTGCGATTC GATAACGACGCTGCAAGTCCTCGCATGGTACCCCGTGCTCCAT GGATGGAGCAGGAAGGAAGTGAATATTGGGACCGCGAAACA CGGAGCGCACGCGATACCGCCCAGATTTTCAGGGTGAACCTC CGTACCCTGAGGGGCTACTATAATCAGAGCGAGGCCGGCTCC CATACTTTGCAGTGGATGCACGGGTGCGAGCTGGGACCCGAT GGCCGTTTTCTGCGGGGTTACGAGCAGTTCGCCTACGACGGC AAGGACTATCTGACACTGAACGAAGATCTCAGGAGCTGGACA GCTGTCGACACCGCTGCCCAGATAAGTGAGCAGAAAAGCAAT GACGCTTCAGAGGCTGAGCATCAGAGGGCATACTTGGAGGAT ACCTGCGTGGAGTGGTTGCATAAGTATCTCGAGAAGGGCAAA GAGACCTTGCTGCATTTGGAGCCCCCCAAAACACATGTGACC CACCACCCCATTAGTGATCATGAAGCCACCCTCCGATGCTGGG | Nucleotide sequence encoding HLA-E protein |
| | CCCTCGGATTCTATCCAGCCGAGATTACTCTGACATGGCAACA GGATGGAGAGGGTCATACCCAGGACACCGAGCTGGTCGAGA CCCGTCCCGCTGGGGACGGTACATTCCAGAAGTGGGCCGCAG TGGTGGTCCCTAGCGGCGAAGAGCAGCGATACACATGTCATG TGCAGCATGAAGGTCTGCCTGAACCTGTTACCCTGCGGTGGA AACCTGCATCTCAGCCCACTATCCCTATTGTGGGCATCATCGCT GGCCTCGTATTGTTGGGAAGCGTGGTGTCAGGCGCTGTGGTG GCTGCTGTGATCTGGCGGAAGAAAAGCTCCGGAGGCAAGGG CGGGTCATATTCTAAGGCAGAATGGTCCGATAGCGCTCAGGGT AGTGAGTCTCACTCTCTG | |
| 54 | NSFTKLSIEPAFTPGPNIELQKDSDCCSCQEKWVGYRCNCYFISS EQKTWNESRHLCASQKSSLLQLQNTDELDFMSSSQQFYWIGLS YSEEHTAWLWENGSALSQYLFPSFETFNTKNCIAYNPNGNALDE SCEDKNRYICKQQLILEPKSCDKTHTCPPCPAPELLGGPSVFLFPP KPKDTLMISRTPEVTCVVVDVSHEDPEVKFNWYVDGVEVHNA KTKPREEQYNSTYRVVSVLTVLHQDWLNGKEYKCKVSNKALP APIEKTISKAKGQPREPQVYTLPPSRDELTKNQVSLTCLVKGFYP SDIAVEWESNGQPENNYKTTPPVLDSDGSFFLYSKLTVDKSRWQ QGNVFSCSVMHEALHNHYTQKSLSLSPGK | Recombinant CD94 extracellular region Fc fusion protein |

The present disclosure is described in detail by examples below. In the examples or test examples, the experimental methods without specifying the specific conditions were carried out under conventional conditions.

The embodiments of the present disclosure are explained below with reference to the examples. Those skilled in the art will appreciate that the following examples are merely used for illustrating the present disclosure and should not be construed as limiting the scope of the present disclosure. Where specific techniques or conditions are not specified in the examples, they are conducted according to technologies or conditions described in the document in the art or according to the product manual. The reagents or instruments used without indicating manufacturers are all conventional products purchasable in the market.

### Example 1: Preparation of anti-human CD94 hybridoma monoclonal antibody

### 1.1 Hybridoma cell screening

This example was used to obtain murine monoclonal antibodies against human CD94, in which the purified recombinant CD94 extracellular region Fc fusion protein (CD94-Fc) (a fusion protein of recombinant CD94 extracellular region and Fc, having an amino acid sequence as set forth in SEQ ID NO: 54), was used as an immunizing antigen to immunize the C57b1/6 mice (9 weeks old, purchased from Shanghai SLAC, weighing about 20 g).

Mice were intraperitoneally immunized three times with a purified antigen and complete Freund's adjuvant, and an immune response was detected after blood collection via the tail veins. The sera were screened by ELISA and flow cytometry using conventional operating procedures to obtain mice with anti-human CD94 immunoglobulin. Splenocytes were taken from mice with the highest anti-CD94 immunoglobulin titer and fused with mouse myeloma SP2/0 cells (ATCC No. CRL-1581). The fused hybridoma cells were subjected to antibody screening to obtain mouse monoclonal antibodies. The specific operations were as follows.

The cells were resuspended in a HAT complete medium (RPMI-1640 medium containing 20% FBS, 1×HAT, and 1×OPI), dispensed into 96-well cell culture plates, and incubated at 37°C and 5% CO₂. On day 5 post-fusion, 50 µL of the HAT complete medium was added to each well. Form day 7 to day 8 post-fusion, the medium was completely replaced with the HT complete medium (RPMI-1640 medium containing 20% FBS, 1×HT, and 1×OPI) at 200 µL/well, based on a cell growth density.

On day 10 to day 11 post-fusion, flow cytometry-based binding assays were performed based on the cell growth density. The medium in positive wells was replaced and cells were transferred into 24-well plates in time for scaled-up culture based on a cell density. The cell lines transferred into 24-well plates were retested, followed by cryopreservation and first subcloning. The screened positive clones from in first subcloning were cryopreserved, followed by second subcloning. The screened positive clones in the second subcloning were cryopreserved for protein expression. The antibodies were further prepared by means of a serum-free cell culture method and purified by protein G affinity chromatography for subsequent functional activity assays.

### 1.2 Sequencing of hybridoma cell

The total number of candidate hybridoma cells screened in the above section 1.1 was cultured to 106 cells. The cells were collected by centrifugation at 800 rpm for 10 minutes, and total RNA was extracted using a Trizol kit (Invitrogen). The total RNA was used as a template for reverse transcription to synthesize a cDNA library (Invitrogen), and cDNA was used as a template for Polymerase Chain Reaction, PCR, amplification of nucleic acid sequences corresponding to CD94 antibody variable regions of the hybridoma cells. The primer sequences used in the PCR amplification reaction were complementary to the first framework region of the antibody variable region or a signal peptide region and a constant region (specific sequences were selected with reference to Larrick, J.W., et al., (1990) Scand. J. Immunol., 32, 121-128 and Coloma, J.J. et al., (1991) BioTechniques, 11, 152-156). The PCR amplification was carried out in a 50 µL reaction system, and 2 µL of cDNA, 5 µL of 10×PCR buffer, 2 µL of each of upstream and downstream primers (5 µM), 2 µL of dNTP, 1 µL of Taq enzyme (Takara, Ex Taq), and 38 µL of H₂O were added. Pre-denaturation was performed at 95°C for 5 minutes, and temperature cycling was performed for the PCR amplification. The reaction conditions included denaturation at 94°C for 30 s, annealing at 58°C for 45 s and extension at 72°C for 50 s for a total of 32 cycles, and then extension at 72°C for 7 minutes. After sequencing the amplified product, the sequences of the heavy chain variable region (SEQ ID NO: 23) and the light chain variable region (SEQ ID NO: 24) of the anti-CD94 mouse monoclonal antibody were obtained.

### Example 2: Flow cytometry binding assay of CD94 chimeric antibody

The flow cytometry assay was used to detect the binding properties of the CD94 chimeric antibody. A CD94 protein and an NKG2A protein (293T-CD94/NKG2A) were overexpressed in 293T cells (ATCC No. CRL-3216). The intensity of the signal after the addition of the antibody was used to determine the binding properties of the chimeric antibody to CD94.

### 2.1 Construction of 293T cell overexpressing human CD94

HEK293T cells were plated into six-well plates at 5×10⁵ cells/well and cultured overnight in DMEM medium without double antibiotics (penicillin-streptomycin). Before transfection, the culture medium was discarded, and 1 mL of fresh DMEM medium without double antibiotics was added. pLVX-EF1a-CD94-IRES-puro vector (a coding sequence (SEQ ID NO: 41) of the human CD94 protein (SEQ ID NO: 40) was inserted between EcoRI and BamHI sites of the pLUX-EF1a-IRES-puro vector), or pLVX-EF1a-NKG2A-IRES-puro (a coding sequence (SEQ ID NO: 43) of a NKG2Aprotein (SEQ ID NO: 42) was inserted between the EcoRI and BamHI sites of the pLUX-EF1a-IRES-puro vector) were mixed with pMD2G and psPAX2 vectors (3 µg in total) at a ratio of 2:1:1 in 200 µL of serum-free DMEM medium, followed by an addition of 12 µg of polyetherimide (PEI, Polysciences, Inc.). After uniformly mixing, the mixture was left to stand for 16 minutes, and then all the liquid was added into a six-well plate containing HEK293T cells. At 6 h of culture, the culture medium was removed, and fresh complete DMEM medium was added for cultivation. At 48 h post-transfection, the cell culture supernatant was collected and filtered through a 0.45 µm filter (Millipore) to obtain the viral supernatant. The entire viral supernatant was added to a 6-well plate containing 1×10⁴ 293T cells, and polybrene (Sigma) at a final concentration of 4 µg/mL was added and cultured for 12 hours. The supernatant was then removed, and fresh complete DMEM medium was added. The obtained cells were the 293T-CD94/NKG2A cells.

### 2.2 Binding activity assay of chimeric antibody CD94

The CD94 chimeric antibody 15C10-hIgG1mut used in this section was expressed in the inventors' laboratory and had a heavy chain amino acid sequence as set forth in SEQ ID NO: 33 and a light chain amino acid sequence as set forth in SEQ ID NO: 34. In addition, the blocking NKG2A antibody huZ270-hIgG1mut used was also expressed in the inventors' laboratory and the heavy chain amino acid sequence thereof is as set forth in SEQ ID NO: 44 and the light chain amino acid sequence thereof is as set forth in SEQ ID NO: 45.

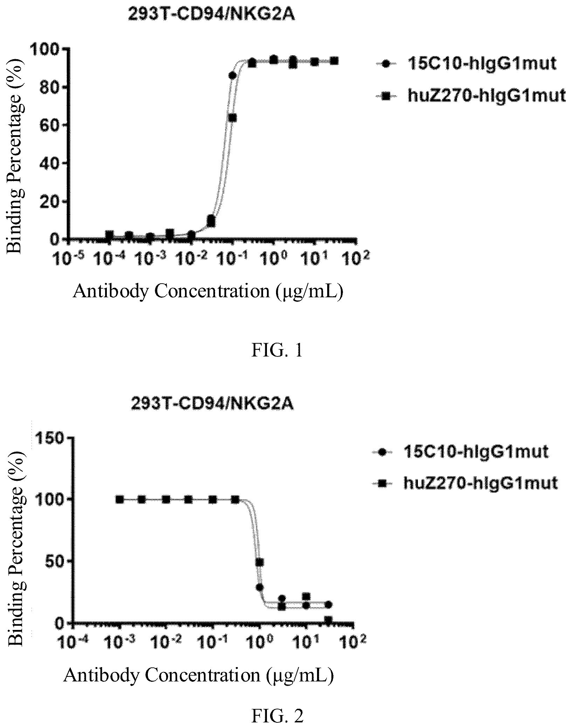

The 293T-CD94/NKG2A cells were diluted to 2×10⁶ /mL with PBS and aliquoted to a 1.5 mL EP tube at a volume of 100 µL/tube. 10 µL/tube of mouse serum was added to the tube, and blocking was conducted at 4°C for 30 minutes. The CD94 chimeric antibody 15C10-hIgG1mut or the blocking NKG2A antibody huZ270-hIgG1mut at different concentration gradients were added separately and incubated at 4°C for 30 minutes. 1 mL of PBS was added to the EP tube, and the mixture was centrifuged at 4°C, 3500 rpm for 5 minutes. The supernatant was removed, and the cells were washed again with PBS. After centrifugation, the supernatant was removed, and the cells were resuspended with 100 µL/tube of PBS. 1 µL/tube of Alexa-647-conjugated goat anti-human Fc secondary antibody (Biolegend) was added, and the samples were incubated at 4°C in the dark for 30 minutes. The cells were washed twice with PBS and pelleted by centrifugation, and the supernatant was removed. The cells were resuspended with 200 µL/tube of PBS and detected by flow cytometry. The results were shown in FIG. 1, and further demonstrated that the 15C10-hIgG1mut chimeric antibody of the present disclosure can bind to CD94.

### Example 3: Assay of blocking capability of CD94 chimeric antibody

The CD94 antibody blocks the binding of CD94/NKG2A and its ligand HLA-E by binding to an extracellular region of the CD94 protein. A flow cytometry assay was used to detect a blocking effect of the CD94 antibody on HLA-E binding to 293T-CD94/NKG2A cells.

The 293T-CD94/NKG2A cells (same as Example 2) were diluted to 2×10⁶ /mL with PBS and aliquoted to a 1.5 mL EP tube at a volume of 100 µL/tube. 10 µL/tube of mouse serum was added to each tube, and blocking was conducted at 4°C for 30 minutes. The above-mentioned CD94 chimeric antibody 15C10-hIgG1mut and blocking NKG2A antibody huZ270-hIgG1mut were added separately and incubated at 4°C for 30 minutes. 0.1 µg/tube of APC-labeled HLA-E tetramer protein was added, and the samples were incubated at 4°C for 30 minutes. The cells were washed twice with PBS, after centrifugation, the supernatant was removed. The cells were resuspended with 200 µL/tube PBS and detected using a flow cytometry. The results were shown in FIG. 2. It can be observed that the CD94 antibody of the present disclosure can block the binding of HLA-E to CD94/NKG2A and had a blocking capability comparable to that of the NKG2A antibody huZ270.

### Example 4: Humanization of murine anti-human CD94 monoclonal antibody

Based on Examples 1 and 2, by aligning the IMGT human antibody heavy and light chain variable region germline gene database and using MOE software, the germline genes of heavy or light chain variable regions with high homology to the murine monoclonal antibody were selected as templates, respectively, and the CDRs of the murine monoclonal antibody were grafted into corresponding human templates to form a variable region sequence arranged in the order of FR1-CDR1-FR2-CDR2-FR3-CDR3-FR4. Amino acid residues were identified and annotated using the Kabat numbering system.

In order to maintain the conformation of CDR region, based on the three-dimensional structure of the murine antibody, the residues on a VL and VH binding interface, the residues close to the CDRs and buried within a protein, and the residues directly interacting with the CDRs were subjected to back mutations to ensure that activity of the variable regions would not be affected, and thus to obtain the humanized antibody. The sequence of the heavy chain variable region of the humanized CD94 antibody was as set forth in SEQ ID NO: 25, and the heavy chain variable region thereof was as set forth in SEQ ID NO: 26.

### Example 5: Flow cytometry binding assay of CD94 chimeric antibody and humanized CD94 antibody

CD94 protein and NKG2A protein (293T-CD94/NKG2A) were overexpressed in 293T cells (ATCC). An ELISA assay was used to detect the binding properties of the CD94 chimeric antibody and the humanized CD94 antibody to 293T-CD94/NKG2A cells.

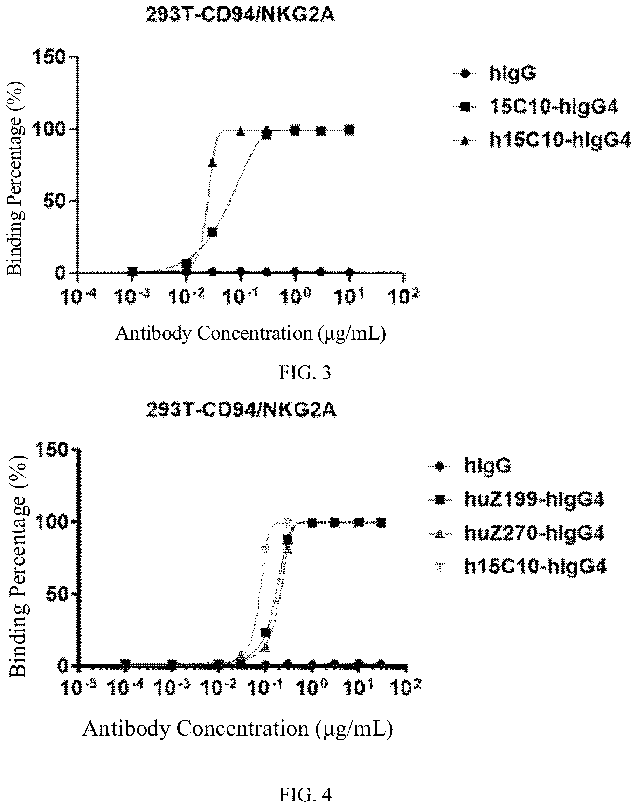

The 293T-CD94/NKG2A cells overexpressing the CD94 protein and the NKG2A protein that were obtained in Example 2 were diluted to 2×10⁶ cells/mL with PBS and aliquoted into a 1.5 mL EP tube at a volume of 100 µL/tube. 10 µL/tube of mouse serum was added to each tube, and blocking was conducted at 4°C for 30 minutes. The CD94 chimeric antibody 15C10-hIgG4 (expressed in the inventors' laboratory and having a heavy chain sequence as set forth in SEQ ID NO: 36 and a light chain sequence as set forth in SEQ ID NO: 37), humanized antibody h15C10-hIgG4 (expressed in the inventors' laboratory and having a heavy chain sequence as set forth in SEQ ID NO: 38 and a light chain sequence as set forth in SEQ ID NO: 39) at gradient concentrations (3-fold dilution, the highest final concentration was 10 µg/mL) were added separately and incubated at 4°C for 30 minutes. 1 mL of PBS was added to the EP tube, and the EP tube was centrifuged at 3500 rpm for 5 minutes at 4°C. The supernatant was removed, and the cells were washed again with PBS. After centrifugation, the supernatant was removed, and the cells were resuspended with 100 µL/tube of PBS. 0.1 µL/tube of Alexa-647-labeled goat anti-human Fc secondary antibody (Biolegend) was added, and the samples were incubated at 4°C in the dark for 30 minutes. The cells were washed twice with PBS, and the supernatant was removed after centrifugation. The cells were resuspended with 200 µL/tube of PBS and detected using a flow cytometry. The results were shown in FIG. 3, and demonstrated that both the humanized antibody and the chimeric antibody of the present disclosure have good binding capabilities, and the humanized antibody demonstrated a superior binding capacity to the chimeric antibody.

### Example 6: ELISA binding assay of Human CD94 antibody

CD94 protein and NKG2A protein (293T-CD94/NKG2A) were overexpressed in 293T cells (ATCC). An ELISA assay was used to detect the binding properties of the humanized CD94 antibody and the NKG2A antibody that were obtained in the above example to 293T-CD94/NKG2A cells.

The 293T-CD94/NKG2A cells overexpressing the CD94 protein and the NKG2A protein that were obtained in Example 2 were diluted to 2×10⁶ cells/mL with PBS and aliquoted into a 1.5 mL EP tube at a volume of 100 µL/tube. 10 µL/tube of mouse serum was added to each tube, and blocking was conducted at 4°C for 30 minutes. The humanized antibody h15C10-hIgG4 (same as Example 5), non-blocking NKG2A antibody huZ199-hIgG4 (expressed in inventors'laboratory and having a heavy chain sequence as set forth in SEQ ID NO: 46 and a light chain sequence as set forth in SEQ ID NO: 47), blocking NKG2A antibody huZ270-hIgG4 (expressed in the inventors' laboratory and having a heavy chain sequence as set forth in SEQ ID NO: 48 and a light chain sequence as set forth in SEQ ID NO: 49), or control antibody hIgG4 at gradient concentrations (3-fold dilution, the highest final concentration was 30 µg/mL) were added separately and incubated at 4°C for 30 minutes. 1 mL of PBS was added to the EP tube, and the EP tube was centrifuged at 3500 rpm for 5 minutes at 4°C. The supernatant was removed, and the cells were washed again with PBS. After centrifugation, the supernatant was removed, and the cells were resuspended with 100 µL/tube of PBS. 0.1 µL/tube of Alexa-647-conjugated goat anti-human Fc secondary antibody (Biolegend) was added, and the samples were incubated at 4°C in the dark for 30 minutes. The cells were washed twice with PBS and the supernatant was removed after centrifugation. The cells were resuspended with 200 µL/tube of PBS and detected using a flow cytometry. The results were shown in FIG. 4, and demonstrated that the humanized CD94 antibody exhibited a superior binding capacity to 293T-CD94/NKG2A cells compared to the NKG2A antibody huZ199 and huZ270.

### Example 7: Assay of blocking capacity of humanized CD94 antibody

The CD94 antibody blocks binding of CD94/NKG2A and its ligand HLA-E by binding to an extracellular region of the CD94 protein. A flow cytometry assay was used to detect the blocking effect of the CD94 antibody on HLA-E binding to 293T-CD94/NKG2A cells.

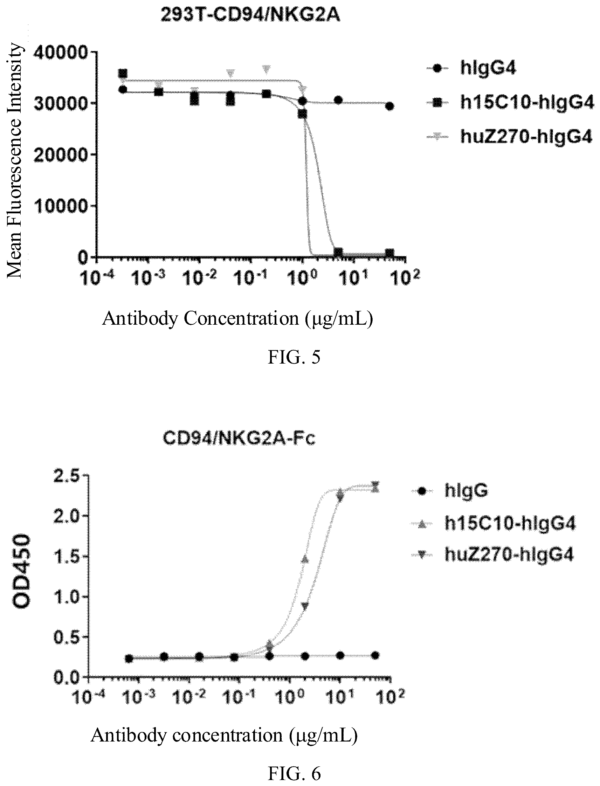

The 293T-CD94/NKG2A cells (same as above) were diluted to 2×10⁶ /mL with PBS and aliquoted to a 1.5 mL EP tube at a volume of 100 µL/tube. 10 µL/tube of mouse serum was added to each tube, and blocking was conducted at 4°C for 30 minutes. The CD94 humanized antibody 15C10-hIgG4, NKG2A antibody huZ270-hIgG4, or control antibody hIgG4 used in the above examples were added separately and incubated at 4°C for 30 minutes. 0.1 µg/tube of APC-conjugated HLA-E tetramer protein was added, and the samples were incubated at 4°C for 30 minutes. The cells were washed twice with PBS, and aftercentrifugation, the supernatant was removed. The cells were resuspended with 200 µL/tube PBS and detected using a flow cytometry. Results were shown in FIG. 5, and demonstrated that the CD94 antibody of the present disclosure can effectively block the binding of HLA-E to CD94/NKG2A and had a blocking ability comparable to that of the NKG2A antibody huZ270.

### Example 8: ELISA binding assay of CD94 antibody

An ELISA assay was used to detect the binding properties of the CD94 antibody. A heterodimer protein composed of a CD94 extracellular region Fc fusion protein and an NKG2A extracellular region Fc fusion protein was coated onto a 96-well plate. The intensity of the signal after the addition of the antibody was used to determine the binding properties of the antibody to the CD94 protein.

The heterodimer fusion protein (expressed in inventors' laboratory, the amino acid sequence of CD94-Fcmutknob was set forth in SEQ ID NO: 50 and the amino acid sequence of NKG2A-Fcmuthole was set forth in SEQ ID NO: 51) composed of the CD94 extracellular region Fc fusion protein and the NKG2A extracellular region Fc fusion protein (CD94/NKG2A-Fc) was diluted to 2 µg/mL with PBS buffer, aliquoted to a 96-well plate at a volume of 100 µL/well, and placed at 4°C overnight. The PBS buffer in the 96-well plate was aspirated, and the plate was washed 6 times with PBST (PBS containing 0.1% Tween 20, pH 7.2) buffer, and then 200 µL/well of PBS/10% BSA was added and incubated at 37°C for 2 hours for blocking. The blocking solution was removed, and the plate was washed 6 times with PBST, and then 100 µL/well of humanized CD94 antibody h15C10-hIgG4 (same as in Example 5) and NKG2A antibody huZ270-hIgG4 to be tested diluted to an appropriate concentration with PBST/0.05% BSA were added and incubated at 37°C for 1 hour. The reaction system was removed, and the plate was washed 6 times with PBST, and then 100 µL/well of HRP (horseradish peroxidase)-labeled goat anti-human IgG-Fab secondary antibody was diluted with PBST/0.05% BSA and incubated at 37°C for 1 hour. After washing the plate six times with PBST, 80 µl/well of TMB (tetramethylbenzidine) was added, incubated at room temperature for 3 minutes. The reaction was terminated by adding 80 µl/well of 4 M sulfuric acid. The absorbance was read at 450 nm using an enzyme reader. The results shown in FIG. 6 demonstrated that the CD94 antibody of the present disclosure can bind to the CD94/NKG2A dimer protein and exhibited stronger binding effect than that of the NKG2A antibody huZ270.

### Example 9: CD94 antibody promoting Jurkat T cell activation assay

The reporter cell assay was used to detect the functional activity of CD94 antibody. K56233220-HLA-E cells were co-incubated with Jurkat-NFAT-lucia-CD94/NKG2A cells. The intensity of the chemiluminescence after the addition of the antibody was used to determine the functional activity of the CD94 antibody in promoting T cell activation.

HEK293T cells were plated into six-well plates at 5×10⁵ cells/well and cultured overnight in DMEM medium without double antibiotics. Before transfection, the culture medium was removed, and 1 mL of fresh DMEM medium without double antibiotics was added. pLVX-EF1a-CD94-IRES-puro vector (a coding sequence (SEQ ID NO: 41) of the human CD94 protein (SEQ ID NO: 40) was inserted between EcoRI and BamHI sites of a pLVX-EF1a-IRES-puro vector), or pPLVX-EF1a-NKG2A-IRES-puro (a coding sequence (SEQ ID NO: 43) of a NKG2A protein (SEQ ID NO: 42) was inserted between the EcoRI and BamHI sites of the pLUX-EF1a-IRES-puro vector) were mixed with pMD2G and psPAX2 vectors (3 µg in total) at a ratio of 2:1:1 in 200 µL of a serum-free DMEM medium, followed by an addition of 12 µg of polyetherimide (PEI, purchased from Polysciences, Inc.). After uniformly mixing, the mixture was left to stand for 16 minutes, and then all the liquid was added into a six-well plate containing HEK293T cells. At 6 h of culture, the culture medium was removed, and fresh complete DMEM medium was added for cultivation. At 48 hours post-transfection, the cell culture supernatant was collected and filtered through a 0.45 µm filter (Millipore) to obtain a viral supernatant. The entire viral supernatant were added to a 6-well plate containing 1×10⁴ Jurkat-NFAT-lucia cells (purchased from Invivogen), and polybrene (Sigma) was added at a final concentration of 4 µg/mL and cultured for 12 hours. The supernatant was then removed and fresh complete IMDM medium was added. The obtained cells were the Jurkat-NFAT-lucia-CD94/NKG2A cells.

HEK293T cells were plated into six-well plates at 5×10⁵ cells/well and cultured overnight in DMEM medium without double antibiotics. Before transfection, the culture medium was removed, and 1 mL of fresh DMEM medium without double antibiotics was added. pLVX-EF1a-CD94-IRES-puro vector (a coding sequence (SEQ ID NO: 52) of an HLA-E protein (SEQ ID NO: 52) was inserted between EcoRI and BamHI sites of the pLUX-EF1a-IRES-puro vector) was mixed with pMD2G and psPAX2 vectors (3 µg in total) at a ratio of 2:1:1 in 200 µL of a serum-free DMEM medium, followed by an addition of 12 µg of polyetherimide (PEI, purchased from Polysciences, Inc.). After uniformly mixing, the mixture was left to stand for 16 minutes, and then all the liquid was added into a six-well plate containing HEK293T cells. After 6 h of culture, the culture medium was removed, and a fresh complete DMEM medium was added for cultivation. At 48 hours post-transfection, the cell culture supernatant was collected and filtered through a 0.45 µm filter (Millipore) to obtain a viral supernatant. The entire viral supernatant was added to a 6-well plate containing 1×10⁴ K56233220 cells, and polybrene (Sigma) at a final concentration of 4 µg/mL was added and cultured for 12 hours. The supernatant was then removed, and fresh complete IMDM medium was added. The obtained cells were the K56233220-HLA-E cells.
(1) Jurkat-NFAT-lucia-CD94/NKG2A cells were diluted to 1×10⁵/mL with complete IMDM medium and added to a 96-well plate at 100 µL/well.
(2) The CD94 humanized antibody h15C10-hIgG4, the NKG2A antibody huZ270-hIgG4, and the control antibody hIGg were diluted into a series of gradients with complete IMDM medium and added to a 96-well plate at 20 µL/well.
(3) The K56233220-HLA-E cells were diluted to 7.5×10⁵/mL with complete IMDM medium and added to a 96-well plate at 80 µL/well.
(4) After culturing in an incubator at 37°C with 5% CO₂ for 24 hours, 50 µL of the supernatant was aspirated, and 50 µL of substrate was added to detect chemiluminescence.

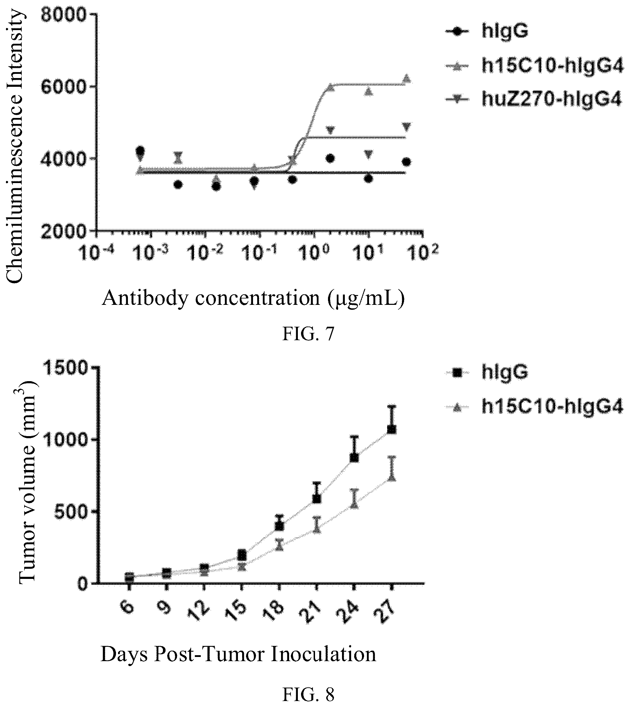

The results were shown in FIG. 7, and further demonstrated that the CD94 antibody of the present disclosure can promote the activation of the Jurkat-NFAT-lucia-CD94/NKG2A T cells and had stronger functional activation than that of the NKG2A antibody huZ270.

### Example 10: Anti-cancer effect of humanized CD94 antibody in mice

An in vivo efficacy assay was used to detect the anti-cancer function of the affinity-matured humanized CD94 antibody h15C10-hIgG4 obtained in Example 6 in promoting immune-reconstituted mice.
(1) On day -1, human PBMC (purchased from Shanghai Saily Biotechnology Co., Ltd.) was infused into NCG mice (purchased from GemPharmatech Co., Ltd) via the tail vein at a dose of 1×10⁷ cells/mouse.
(2) On day 0, the NCG mice were subcutaneously implanted with 1×10⁶ tumor cells in the right flank, and the mice were randomly divided into groups.
(3) On days 0, 3, 6, and 9, the humanized antibody h15C10-hIgG4 and the control antibody hIgG were intraperitoneally injected into the mice at 250 µg/mouse.
(4) A tumor volume was measured every 3 days after the injection of the above antibodies.

Results were shown in FIG. 8, which demonstrates that the CD94 antibody of the present disclosure can effectively promote the anti-cancer effect of PBMC.

Based on the above assay results, the antibodies obtained in the present disclosure can bind to CD94, block the interaction between CD94/NKG2A and HLA-E, and promote the anti-cancer effect of the immune cells.

In the present description, descriptions of the reference terms such as "one embodiment", "some embodiments", "examples", "specific examples" and "some examples" mean that, specific features, structures, materials or characteristics described in combination with the embodiments or examples are included in at least one embodiment or example of the present disclosure. In the present description, schematic expressions of the above terms are unnecessarily specified at the same embodiment or example. Moreover, the specific features, structures, materials or characteristics described herein may be in combination in any of or more embodiments or examples in an appropriate mode. In addition, in absence of mutual contradiction, different embodiments or examples described in the present description and features of the different embodiments or examples may be in integration and combination by those skilled in the art.

## Claims

1. An antibody or antigen-binding fragment, comprising:
a heavy chain CDR1, a heavy chain CDR2, and a heavy chain CDR3; and
a light chain CDR1, a light chain CDR2, and a light chain CDR3, wherein:
an amino acid sequence of the heavy chain CDR1 has at least 80% homology to a sequence as set forth in SEQ ID NO: 1;
an amino acid sequence of the heavy chain CDR2 has at least 80% homology to a sequence as set forth in SEQ ID NO: 2;
an amino acid sequence of the heavy chain CDR3 has at least 80% homology to a sequence as set forth in SEQ ID NO: 3;
an amino acid sequence of the light chain CDR1 has at least 80% homology to a sequence as set forth in SEQ ID NO: 4;
an amino acid sequence of the light chain CDR2 has at least 80% homology to a sequence as set forth in SEQ ID NO: 5; and
an amino acid sequence of the light chain CDR3 has at least 80% homology to a sequence as set forth in SEQ ID NO: 6.

2. The antibody or antigen-binding fragment thereof according to claim 1, comprising:
a heavy chain variable region as set forth in SEQ ID NO: 23 or SEQ ID NO: 25; and/or
a light chain variable region as set forth in SEQ ID NO: 24 or SEQ ID NO:26.

3. The antibody or antigen-binding fragment thereof according to claim 1, comprising:
a heavy chain variable region as set forth in SEQ ID NO: 23 and a light chain variable region as set forth in SEQ ID NO: 24; or
a heavy chain variable region as set forth in SEQ ID NO: 25 and a light chain variable region as set forth in SEQ ID NO: 26.

4. The antibody or antigen-binding fragment thereof according to any one of claims 1 to 3, comprising at least one of a heavy chain constant region and a light chain constant region, wherein at least part of the at least one of the heavy chain constant region and the light chain constant region is from at least one of a humanized antibody, a primate-derived antibody, a murine antibody, or a mutant thereof.

5. The antibody or antigen-binding fragment thereof according to claim 4, wherein the light chain constant region and the heavy chain constant region are each from:
a murine IgG antibody or a mutant thereof; or
a humanized IgG antibody or a mutant thereof.

6. The antibody or antigen-binding fragment thereof according to claim 5, wherein the light chain constant region and the heavy chain constant region are each from:
a murine IgG1 antibody, a murine IgG4 antibody, or a mutant thereof; or
a human IgG1 antibody, a human IgG4 antibody, or a mutant thereof.

7. The antibody or antigen-binding fragment thereof according to claim 1, comprising:
a heavy chain constant region having an amino acid sequence as set forth in SEQ ID NO: 27, SEQ ID NO:29, or SEQ ID NO:31, and/or a light chain constant region having an amino acid sequence as set forth in SEQ ID NO: 28, SEQ ID NO:30, or SEQ ID NO:32;
a heavy chain constant region having an amino acid sequence as set forth in SEQ ID NO: 27, and a light chain constant region having an amino acid sequence as set forth in SEQ ID NO: 28;
a heavy chain constant region having an amino acid sequence as set forth in SEQ ID NO: 29, and a light chain constant region having an amino acid sequence as set forth in SEQ ID NO: 30; or
a heavy chain constant region having an amino acid sequence as set forth in SEQ ID NO:31, and a light chain constant region having an amino acid sequence as set forth in SEQ ID NO:32.

8. The antibody or antigen-binding fragment thereof according to claim 1, comprising:
a heavy chain having an amino acid sequence as set forth in any one of SEQ ID NO: 33, SEQ ID NO: 35, SEQ ID NO: 36, and SEQ ID NO: 38; and
a light chain having an amino acid sequence as set forth in any one of SEQ ID NO: 34, SEQ ID NO: 37, and SEQ ID NO: 39.

9. The antibody or antigen-binding fragment thereof according to claim 1, comprising:
a heavy chain having an amino acid sequence as set forth in SEQ ID NO: 33, and a light chain having an amino acid sequence as set forth in SEQ ID NO: 34;
a heavy chain having an amino acid sequence as set forth in SEQ ID NO: 35, and a light chain having an amino acid sequence as set forth in SEQ ID NO: 37;
a heavy chain having an amino acid sequence as set forth in SEQ ID NO: 36, and a light chain having an amino acid sequence as set forth in SEQ ID NO: 37; or
a heavy chain having an amino acid sequence as set forth in SEQ ID NO: 38, and a light chain having an amino acid sequence as set forth in SEQ ID NO: 39.

10. The antibody or antigen-binding fragment thereof according to claim 1, comprising a monoclonal antibody or a polyclonal antibody, wherein:
the monoclonal antibody comprises at least one of a full-length antibody, Fv, single-chain antibody, Fab, single-domain antibody, and a minimal recognition unit; and
the antibody or antigen-binding fragment thereof is capable of binding to an amino acid sequence as set forth in SEQ ID NO: 40.

11. A nucleic acid molecule, encoding the antibody or antigen-binding fragment thereof according to any one of claims 1 to 10.

12. An expression vector, carrying the nucleic acid molecule according to claim 11.

13. A recombinant cell, carrying the nucleic acid molecule according to claim 11 or the expression vector according to claim 12, or being capable of expressing the antibody or antigen-binding fragment thereof according to any one of claims 1 to 10.

14. The recombinant cell according to claim 13, wherein:
the recombinant cell is obtained by introducing the expression vector according to claim 12 into a host cell; and
the recombinant cell is a eukaryotic cell.

15. The recombinant cell according to claim 14, wherein the recombinant cell is a mammalian cell.

16. A composition, comprising the antibody or antigen-binding fragment thereof according to any one of claims 1 to 10, the nucleic acid molecule according to claim 11, the expression vector according to claim 12, or the recombinant cell according to any one of claims 13 to 15.

17. A medicament, comprising the antibody or antigen-binding fragment thereof according to any one of claims 1 to 10, the nucleic acid molecule according to claim 11, the expression vector according to claim 12, the recombinant cell according to any one of claims 13 to 15, or the composition according to claim 16.

18. A kit, comprising the antibody or antigen-binding fragment thereof according to any one of claims 1 to 10, the nucleic acid molecule according to claim 11, the expression vector according to claim 12, or the recombinant cell according to any one of claims 13 to 15.

19. Use of the antibody or antigen-binding fragment thereof according to any one of claims 1 to 10, the nucleic acid molecule according to claim 11, the expression vector according to claim 12, the recombinant cell according to any one of claims 13 to 15, or the composition according to claim 16 in the preparation of a medicament for preventing and/or treating a CD94-mediated disease,
wherein the CD94-mediated disease comprises a transplant rejection, an autoimmune disease, an infectious disease, and cancer, wherein:
the autoimmune disease comprises at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis; and
the cancer comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

20. Use of the antibody or antigen-binding fragment thereof according to any one of claims 1 to 10, the nucleic acid molecule according to claim 11, the expression vector according to claim 12, or the recombinant cell according to any one of claims 13 to 15 in the preparation of a kit for detecting CD94.

21. Use of the antibody or antigen-binding fragment thereof according to any one of claims 1 to 10, the nucleic acid molecule according to claim 11, the expression vector according to claim 12, the recombinant cell according to any one of claims 13 to 15, the composition according to claim 16, or the medicament according to claim 17 in the treatment or prevention of a CD94-mediated disease.

22. Use of the antibody or antigen-binding fragment thereof according to any one of claims 1 to 10, the nucleic acid molecule according to claim 11, the expression vector according to claim 12, or the recombinant cell according to any one of claims 13 to 15 in the diagnosis, staging or assessment of a CD94-mediated disease.

23. A method for treating or preventing a CD94-mediated disease, the method comprising:
administering to a subject at least one of:
the antibody or antigen-binding fragment according to any one of claims 1 to 10;
the nucleic acid molecule according to claim 11;
the expression vector according to claim 12;
the recombinant cell according to any one of claims 13 to 15;
the composition according to claim 16; and
the medicament according to claim 17.

24. A method for assessing a prognosis of a CD94-mediated disease, the method comprising:
detecting CD94 in a sample to be tested using at least one of the antibody or antigen-binding fragment thereof according to any one of claims 1 to 10, the nucleic acid molecule according to claim 11, the expression vector according to claim 12, and the recombinant cell according to any one of claims 13 to 15;
determining, based on a detection result of CD94, a content of CD94 in the sample to be tested; and
determining, based on the content of CD94 in the sample to be tested before or after treatment, a prognostic effect of the CD94-mediated disease.

25. The use according to claims 21 and 22 or the method according to claims 23 and 24, wherein the CD94-mediated disease comprises a transplant rejection, an autoimmune disease, an infectious disease, and cancer, wherein:
the autoimmune disease comprises at least one of systemic lupus erythematosus, rheumatoid arthritis, systemic vasculitis, scleroderma, dermatomyositis, autoimmune hemolytic anemia, thyroid autoimmune disease, ulcerative colitis, chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritis syndrome, pemphigus vulgaris, pemphigoid, primary biliary cirrhosis, multiple sclerosis, and acute idiopathic polyneuritis; and
the cancer comprises at least one of lung cancer, liver cancer, ovarian cancer, cervical cancer, skin cancer, bladder cancer, colon cancer, breast cancer, glioma, renal cancer, gastric cancer, esophageal cancer, oral squamous cell carcinoma, and head-and-neck cancer.

26. The method according to claim 24, wherein:
the sample to be tested is from a patient suffering from the CD94-mediated disease before or after the treatment;
the sample to be tested comprises at least one of blood, saliva, sweat, tissue, cell, blood, serum, plasma, feces, and urine; and
a decrease in the content of CD94 in the sample to be tested of the patient suffering from the CD94-mediated disease after the treatment is an indication of a good prognosis for the patient.
